(19) Europäisches Patentamt
European Patent Office
Office européen des brevets

(11) Numéro de publication : **0 424 260 B1**

(12) # FASCICULE DE BREVET EUROPEEN

(45) Date de publication du fascicule du brevet : **15.12.93 Bulletin 93/50**

(51) Int. Cl.$^5$ : **A61K 7/06,** A61K 7/48

(21) Numéro de dépôt : **90402926.1**

(22) Date de dépôt : **18.10.90**

(54) **Utilisation en cosmétique d'une dispersion aqueuse à base d'organopolysiloxanes et d'un copolymère acrylate d'ammonium/acrylamide réticulé.**

(30) Priorité : **20.10.89 FR 8913787**

(43) Date de publication de la demande : **24.04.91 Bulletin 91/17**

(45) Mention de la délivrance du brevet : **15.12.93 Bulletin 93/50**

(84) Etats contractants désignés : **AT BE CH DE DK ES FR GB GR IT LI NL SE**

(56) Documents cités :
**EP-A- 0 116 207**
**EP-A- 0 173 033**
**EP-A- 0 260 641**
**GB-A- 2 014 584**
**CHEMICAL ABSTRACTS, vol. 99, no. 16, octobre 1983, page 342, résumé no. 128150k, Columbus, Ohio, US; Anon.: "Cosmetic creams and lotions", & RES. DISCL. 1983. 232, 276-7**

(73) Titulaire : **L'OREAL**
**14, Rue Royale**
**F-75008 Paris (FR)**

(72) Inventeur : **Dubief, Claude**
**9, rue Edmond Rostand**
**F-78150 Le Chesnay (FR)**
Inventeur : **Cauwet, Danièle**
**53, rue de Charonne**
**F-75011 Paris (FR)**

(74) Mandataire : **Casalonga, Axel et al**
**BUREAU D.A. CASALONGA - JOSSE**
**Morassistrasse 8**
**D-80469 München (DE)**

EP 0 424 260 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne l'utilisation en cosmétique, pour le traitement des cheveux ou de la peau et/ou en dermatologie, d'une dispersion aqueuse à base d'organopolysiloxanes et d'un copolymère acrylate d'ammonium/acrylamide réticulé.

On utilise déjà des huiles siliconées en cosmétique comme lubrifiant dans les compositions de traitement des cheveux et de la peau. Il s'agit principalement de polydiméthylsiloxanes.

On connait également des formulations sous forme de spray-aérosol contenant des polydiméthylsiloxanes cycliques, destinées à la fixation des cheveux. Elles sont décrites dans la demande EP 0 116 207.

Afin d'apporter de la douceur aux cheveux ou à la peau, ou encore de faciliter le démêlage des cheveux, on utilise depuis longtemps des polymères ou des tensio-actifs cationiques. Les composés cationiques présentent l'inconvénient, après applications répétées, d'alourdir la chevelure en lui donnant un aspect poisseux ou de produire un effet collant sur la peau.

La demanderesse a découvert, d'une manière surprenante, que l'utilisation d'une dispersion aqueuse à base d'organopolysiloxanes et d'un copolymère acrylate d'ammonium/acrylamide réticulé pour le traitement des cheveux, permet d'obtenir des cheveux brillants, soyeux, légers, dont les propriétés de démêlage et de douceur sont sensiblement améliorées.

L'utilisation de cette dispersion aqueuse dans le traitement de la peau permet également de conférer à celle-ci un toucher doux sans effet collant.

Les dispersions aqueuses utilisées en cosmétique ou dermatologie, selon la présente invention, se répartissent beaucoup plus facilement sur la peau et sur les cheveux que les compositions de l'art antérieur à base de composés cationiques.

La demanderesse a découvert également que les compositions cosmétiques sous forme de dispersion aqueuse, selon la présente invention, étaient remarquablement stables, que leurs propriétés cosmétiques se conservaient même après plusieurs applications successives et plus particulièrement en application non rincée sur les cheveux.

Un objet de l'invention est donc constitué par l'utilisation dans le traitement cosmétique des cheveux ou de la peau et/ou dermatologique, d'une dispersion aqueuse contenant au moins un organopolysiloxane et un copolymère acrylate d'ammonium/acrylamide réticulé.

Un autre objet de l'invention concerne des compositions cosmétiques ou dermatologiques pour le traitement des cheveux ou de la peau, sous forme de dispersions aqueuses.

Un autre objet de l'invention concerne des procédés de traitement cosmétique des cheveux ou de la peau, mettant en oeuvre ces compositions, selon l'application désirée.

D'autres objets de l'invention apparaîtront à la lumière de la description et des exemples qui suivent.

La présente invention a pour objet principal l'utilisation pour le traitement cosmétique des cheveux ou de la peau et/ou dermatologique, d'une dispersion aqueuse, caractérisée par le fait que celle-ci contient au moins dans un milieu aqueux cosmétiquement ou physiologiquement acceptable, un organopolysiloxane et un copolymère acrylate d'ammonium/acrylamide réticulé.

Les organopolysiloxanes utilisés dans les dispersions selon la présente invention, sont des huiles d'organopolysiloxanes ou des solutions organiques de gomme ou de résine d'organosiloxanes.

Parmi les organosiloxanes utilisés conformément à la présente invention, on peut citer à titre non limitatif :

### I. Les silicones volatiles.

Celles-ci possèdent un point d'ébullition compris entre 60°C et 260°C. Parmi ce type de silicones, on cite : (i) les silicones cycliques de 2 à 7 atomes de silicium et de préférence 4 à 5. Il s'agit, par exemple, de l'octaméthylcyclotétrasiloxane vendu sous le nom VOLATILE SILICONE 7207 par UNION CARBIDE ou SILBIONE 70045 V2 par RHONE POULENC, le décaméthylcyclopentasiloxane vendu sous le nom de VOLATILE SILICONE 7158 par UNION CARBIDE, SILBIONE 70045 V5 par RHONE POULENC, ainsi que leurs mélanges.

On cite également les cyclocopolymères du type diméthylsiloxane/méthylalkylsiloxane, tel que la SILICONE VOLATILE FZ 3109 vendue par la Société UNION CARBIDE, de structure chimique :

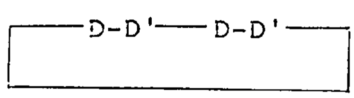

avec

D : 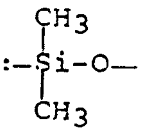

D' : 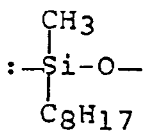

(ii) les silicones volatiles linéaires ayant 3 à 9 atomes de silicium et possédant une viscosité inférieure ou égale à $5.10^{-6} m^2/s$ à 25°C. Il s'agit, par exemple, de l'hexaméthyldisiloxane vendu sous la dénomination SILBIONE 70 041 V 0,65 par la Société RHONE POULENC. Ce type de produit est décrit dans l'article de TODD & BYERS "Volatile silicone fluids for cosmetics", Cosmetics and Toiletries, Vol. 91, Jan 76, p. 27-32.

II. <u>Les silicones non volatiles.</u>

Elles sont constituées principalement par les polyalkylsiloxanes, les polyarylsiloxanes, les polyalkyl arylsiloxanes, les copolymères polyéthersiloxanes modifiés ou non, les gommes et résines de silicone et les polysiloxanes organomodifiés, ainsi que leurs mélanges.

Parmi les polyalkylsiloxanes, on peut citer principalement les polydiméthylsiloxanes linéaires à groupements terminaux triméthylsilyle de viscosité $5.10^{-6}$ à $2,5.10^{-1} m^2/s$ à 25°C et de préférence$10^{-5}$ à 1 $m^2/s$ comme par exemple, et à titre non limitatif :

- les huiles SILBIONE de la série 70 047 et 47 commercialisées par RHONE POULENC comme l'huile 47 V 500 000 ou 70 047 V 300 de RHONE POULENC,
- les huiles de la série 200 de DOW CORNING,
- les Viscasil de la GENERAL ELECTRIC;

et certaines huiles des séries SF de la GENERAL ELECTRIC (SF 96, SF 18).

On cite également les polydiméthylsiloxanes linéaires à groupements terminaux trihydroxysilyle, telles que les huiles de la série 48 Vde RHONE POULENC.

Dans cette classe de polyalkylsiloxanes, on peut également mentionner les polyalkylsiloxanes vendus par la Société GOLDSCHMIDT sous les dénominations ABILWAX 9800 et ABILWAX 9801, qui sont des polyalkyl $(C_1$-$C_{20})$siloxanes.

Parmi les polyalkylarylsiloxanes, on peut citer les polydiméthylphénylsiloxanes, les polydiméthyl diphénylsiloxanes linéaires et/ou ramifiés, de viscosité $10^{-5}$ à $5.10^{-2} m^2/s$ à 25°C, tels que, par exemple :

- l'huile RHODORSIL 763 de RHONE POULENC,
- les huiles SILBIONE 70 641 de RHONE POULENC, telles que l'huile SILBIONE 70 641 V 30 et 70 641 V 200,
- le produit DC 556 Cosmétic Grade Fluid de DOW CORNING,
- l'huile AB12 AV 1000 de GOLDSCHMIDT,
- les silicones des séries PK de BAYER, telles que la PK20,
- les silicones des séries PN, PH de BAYER, comme les PN 1000 et PH 1000,
- certaines huiles des séries SF de GENERAL ELECTRIC, telles que les SF 1250, SF 1265, SF 1154, SF 1023.

Parmi les copolymères polyéthersiloxanes modifiés ou non, on peut citer les copolymères d'oxyde d'éthylène et/ou de propylène avec un diorganosiloxane, tels que par exemple les diméthicone copolyols vendus par la Société DOW CORNING sous la dénomination DC 1248 et l'alkyl$(C_{12})$méthicone copolyol vendu par la Société DOW CORNING sous la dénomination Q2 5200.

On peut citer également les SILWET L 77, L 711, L 722, L 7500 vendus par la Société UNION CARBIDE.

Les gommes de silicone, conformes à la présente invention, sont des polydiorganosiloxanes de forte masse moléculaire comprise entre 200 000 et 1 000 000, utilisés seuls ou en mélange dans un solvant choisi parmi : les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés suivants :

- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)].

On peut citer par exemple, à titre non limitatif, les mélanges suivants :

1/ les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit Q2 1401 vendu par la Société DOW CORNING;

2/ les mélanges formés à partir d'une gomme polydiméthylsiloxane avec une silicone cyclique, tel que le produit SF 1214 SILICONE FLUID de GENERAL ELECTRIC, que est une gomme SE 30 de PM 500.000 solubilisée dans la SF 1202 SILICONE FLUID (décaméthylcyclopentasiloxane).

3/ les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de $20 m^2/s$ et d'une huile SF 96 d'une viscosité de $5.10^{-6}$ $m^2/s$ (15% de gomme SE 30 et 85% d'huile SF 96). Le produit CF 1241 est le mélange d'une gomme SE 30 (33%) et d'une PDMS (67%), ayant une viscosité de $10^{-3}$ $m^2/s$;

4/ les mélanges d'un polydiméthylsiloxane à chaînes latérales polyoxyéthylénées ou polyoxypropylénées (DIMETHICONE COPOLYOL) avec un polydiméthylsiloxane cyclique (CYCLOMETHICONE) tels que le produit Q2 3225 C de la Société DOW CORNING.

Les résines d'organopolysiloxanes utilisables conformément à l'invention sont des systèmes siloxaniques réticulés renfermant les unités :

$R_2SiO_{2/2}$, $RSiO_{3/2}$ et $SiO_{4/2}$, dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination DOW CORNING 593 et les SILICONES FLUID SS 4230 et SS 4267 de la GENERAL ELECTRIC (diméthyl/triméthyl polysiloxane).

Les silicones organomodifiées, conformes à la présente invention, sont des silicones telles que définies précédemment, comportant dans leur structure générale, un ou plusieurs groupements organo-fonctionnels directement fixés sur la chaîne siloxanique ou fixés par l'intermédiaire d'un radical hydrocarboné.

On cite, par exemple, les silicones comportant :

a. des groupements aminés substitués ou non, comme dans la GP4 SILICONE FLUID de GENESEE, la GP 7100 de GENESEE, la Q2 8220 et la DC 929 de DOW CORNING, l'AFL 40 d'UNION CARBIDE.

b. des groupements thiol comme dans les silicones GP 72 A et GP 71 de GENESEE.

c. des groupements carboxylates, comme les produits décrits dans le brevet européen EP 186 507 de CHISSO CORPORATION.

d. des groupements hydroxylés, comme les polyorganosiloxanes à fonction hydroxyalkyle, décrits dans la demande de brevet en France n° FR-85 16334, répondant à la formule suivante :

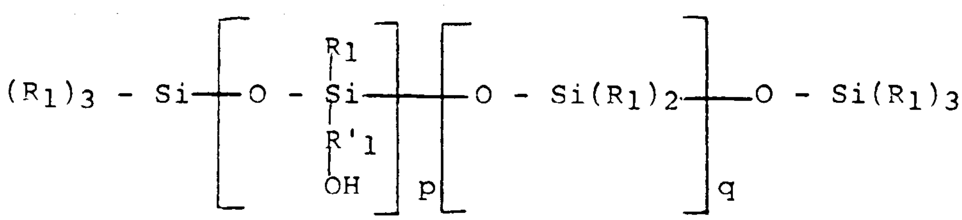

(I)

dans laquelle :

. les radicaux $R_1$, identiques ou différents, sont choisis parmi les radicaux méthyle et phényle, au moins 60% en mole des radicaux $R_1$ étant méthyle;

. le radical $R'_1$ est un chaînon alkylène divalent hydrocarboné en $C_2$-$C_{18}$;

. p est compris entre 1 et 30 inclus;

. q est compris entre 1 et 150 inclus.

e. des groupements alcoxylés comme dans la Silicone copolymer F 755 de SWS SILICONES et les produits ABILWAX 2428, ABILWAX 2434, ABILWAX 2440 de la Société GOLDSCHMIDT.

f. des groupements acyloxyalkyle, comme par exemple les polyorganopolysiloxanes décrits dans la demande de brevet français n° 88 17433, répondant à la formule suivante :

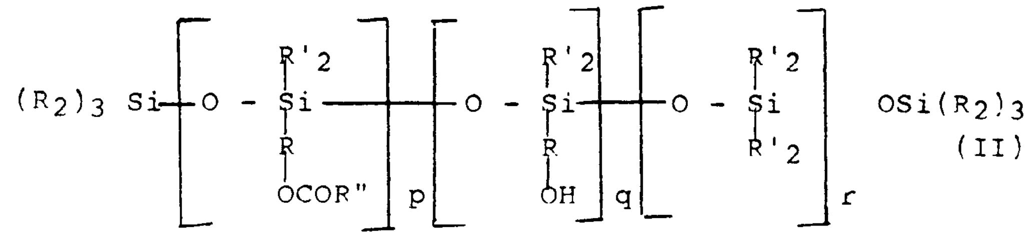

(II)

dans laquelle :

. $R_2$ désigne méthyle, phényle, -COOR", hydroxyle, un seul des $R_2$ par atome de silicium peut être OH;

. $R'_2$ désigne méthyle, phényle, 60% molaire au moins de l'ensemble des radicaux $R_2$ et $R'_2$ est méthyle;

. R" désigne alcoyle ou alcényle an $C_8$-$C_{20}$;

. R désigne un alkylène hydrocarboné divalent, linéaire ou ramifié, en $C_2$-$C_{18}$;

. r est compris entre 1 et 120 inclus;

. p est compris entre 1 et 30;

. q vaut 0 ou est inférieur à 0,5 p, p+q étant compris entre 1 et 30; les polyorganosiloxanes de formule (II) peuvent contenir des groupements

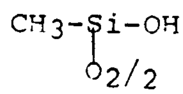

dans des proportions ne dépassant pas 15% de la somme p+q+r.

Ces composés de formule (II) peuvent être préparés par estérification des polyorganosiloxanes à fonction hydroxyalkyle de formule (I) ci-dessus.

L'estérification s'effectue de façon connue, avec un acide R"COOH ou l'anhydride d'acide, à une température comprise entre 100 et 250°C en présence éventuellement d'un catalyseur comme le chlorure d'aluminium ou le chlorure de zinc ou d'un acide fort comme l'acide chlorhydrique ou l'acide sulfurique.

On peut également effectuer une transestérification par chauffage à 100-150°C d'un ester méthylique de formule $R''COOCH_3$ et d'un diorganopolysiloxane de formule (I), en présence d'un catalyseur acide comme l'acide paratoluènesulfonique ou une terre acide de type Montmorillonite (KATALYSATOR KSF/O, vendu par SUD-CHEMIE - A.G. MUNCHEN).

g. des groupements diester et notamment itaconate de diméthyle, comme les huiles à fonction itaconate de diméthyle décrites dans la demande de brevet au Luxembourg n° 87 350 de la demanderesse, répondant à la formule suivante :

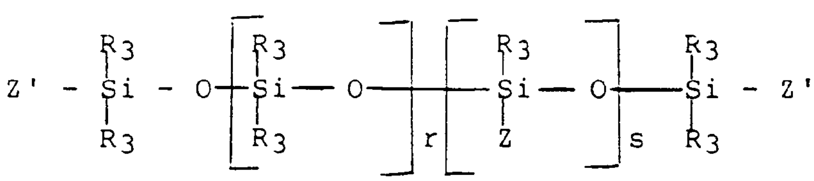

(III)

dans laquelle :

. les radicaux $R_3$, identiques ou différents, désignent un alkyle en $C_1$-$C_{20}$, vinyle, phényle, trifluoro-3,3,3 propyle ou hydroxyle, sous réserve qu'un seul radical $R_3$ peut désigner OH par atome de silicium;

. les symboles Z', identiques ou différents, désignent $R_3$ ou Z;

. Z est choisi parmi les groupes :

$-CH_2-CH-(COOR'_3)-CH_2-COOR'_3$

$-C(CH_3)(COOR'_3)-CH_2-COOR'_3$

. $R'_3$ désigne un radical hydrocarboné saturé, monovalent en $C_1$-$C_{12}$, un alcoxyalkyle monovalent en $C_2$-$C_{12}$, un radical aryle, aralkyle ou alkylaryle en $C_6$-$C_{12}$;

. r est compris entre 0 et 500 inclus;

. s est compris entre 0 et 50 inclus;

. si s est égal à 0, un des symboles Z' vaut Z.

Ces composés peuvent être préparés selon les procédés ci-après.

Au cours d'une première étape ($A_1$), on additionne un diester organique de formule :

$$CH_2 = \underset{\displaystyle CH_2 - COOR'_3}{C} - COOR'_3 \qquad (IIIA)$$

R'$_3$ ayant la même signification qu'à la formule (III), sur un hydrogénoorganosilane de formule :

$$H -(R_3)_a - Si - Cl_{3-a} \qquad (IIIB)$$

$R_3$ ayant la même signification qu'à la formule (III) ci-dessus et a étant égal à 0,1 ou 2.

On obtient ainsi on produit d'addition de formule :

$$Z - (R_3)_a - Si - Cl_{3-a} \qquad (IIIC)$$

dans laquelle $R_3$, Z et a ont les significations indiquées ci-dessus.

L'étape ($A_1$) peut être effectuée en masse ou en solution dans un solvant organique.

La réaction est exothermique. On opère généralement sous reflux du mélange réactionnel à une température comprise entre 60°C at 140°C pendant une durée comprise entre 10 minutes et 3 heures.

On peut, soit couler le silane de formule (IIIB) sur le diester de formule (IIIA) ou vice-versa, ou en même temps.

Il est préférable d'utiliser un excès molaire (de 10 à 50%) de silane de formule (IIIB).

En vue d'augmenter la cinétique de réaction, on opère en présence d'un catalyseur. Les catalyseurs utilisables sont ceux utilisés pour faire la réaction d'hydrosilylation; sont donc en particulier utilisables, les peroxydes organiques, les radiations UV et les catalyseurs à base d'un métal du groupe du platine, en particulier le platine, le ruthénium et le rhodium à la dose de 10 à 500 ppm (calculés en poids de métal) par rapport au poids de silane de formule (IIIB).

En fin de réaction, les produits volatils sont éliminés par distillation sous vide. Le vide de la pompe à eau de 0,1 à 3 KPa est généralement suffisant.

Au cours d'une deuxième étape ($A_2$), on effectue l'hydrolyse (ou la cohydrolyse) et la polycondensation d'un silane de formule (IIIC).

Cette hydrolyse ou cohydrolyse et polycondensation peuvent être effectuées de préférence en phase aqueuse liquide en milieu acide (de préférence HCl) ou en milieu basique (de préférence $NH_4OH$) ou en milieu solvant, dans des conditions similaires à elles de l'hydrolyse des chlorosilanes telles que décrites aux pages 193 à 200 de l'ouvrage de NOLL "CHEMISTRY and TECHNOLOGY OF SILICONES" Academic Press (1968).

La concentration en acide ou en base dans l'eau est comprise généralement entre 10 et 30% en poids. Le milieu d'hydrolyse comporte toujours au moins 2 moles d'eau par mole de silane, généralement de 10 à 100 moles d'eau. L'hydrolyse peut être effectuée en continu ou en discontinu à température ambiante (20°C) ou à une température comprise entre 5 et 90°C. L'hydrolyse peut être effectuée à pression égale ou supérieure à la pression atmosphérique en continu ou en discontinu avec, au moins pour le procédé continu, réinjection d'eau pour maintenir une phase aqueuse constante.

En vue d'obtenir les polymères de formule (III) ou leurs mélanges, on hydrolyse et polycondense les silanes de formule (IIIC) dans laquelle a=1 an présence éventuellement d'un dichlorodiorganosilane de formule :

$$(R_3)_2SiCl_2 \qquad (IIID)$$

dans laquelle $R_3$ a la définition donnée à la formule (III) ci-dessus.

La polycondensation peut être arrêtée par simple neutralisation du milieu réactionnel. Dans ce cas, les polymères de formule (III) obtenus sont bloqués à chacune de leurs extrémités par un groupe hydroxyle (silanol) ou par le motif $(R_3)_2Z\ SiO_{0,5}$ si on utilise le silane $(R_3)_2Z\ SiCl$.

On peut également arrêter la polycondensation an ajoutant on composé organosilicié susceptible de réagir avec les hydroxyles terminaux.

La durée de l'hydrolyse peut être comprise entre quelques secondes et plusieurs heures.

Après hydrolyse, la phase aqueuse est séparée de la phase siloxane par tout moyen physique approprié, généralement par décantation et extraction par un solvant organique comme l'éther isopropylique.

Pour préparer les polymères de formule (III), on peut également selon un deuxième procédé (B), partir du polymère correspondant dans lequel tous les radicaux Z et éventuellement Z', sont des atomes d'hydrogène et par une réaction d'hydrosilylation, additionner un diester de formule (IIIA) ci-dessus.

Le procédé (B) met en oeuvre, comme à l'étape ($A_1$) du procédé (A), une réaction d'hydrosilylation analogue et il est souhaitable d'effectuer cette réaction avec les mêmes catalyseurs que ceux indiqués à l'étape

($A_1$).

Cette réaction peut s'effectuer en masse ou au sein d'un solvant organique à une température comprise entre la température ordinaire (25°C) et 170°C.

Les produits volatils sont éliminés en fin de réaction par distillation sous vide et/ou par extraction.

h. des groupements ammonium quaternaire, comme dans les produits X2 81 08 et X2 81 90, le produit ABIL K 3270 de la Société GOLDSCHMIDT.

i. des groupements amphotères ou bétaïniques, tels que dans le produit vendu par la Société GOLDSCHMIDT sous la dénomination ABIL B 9950.

j. des groupements anioniques de type carboxylique, tels que les groupements alkylcarboxyliques comme ceux présents dans le produit X-22-370/E de la Société SHIN-ETSU; 2-hydroxyalkylsulfonate; 2-hydroxyalkylthiosulfate, tels que les produits vendus par la Société GOLDSCHMIDT sous les dénominations "ABIL S 201" et "ABIL S 255".

Les polyorganosiloxanes particulièrement préférés, selon la présente invention, sont choisis parmi :

1/ les silicones volatiles cycliques, telles que l'octaméthylcylotétrasiloxane et le décaméthylcyclopentasiloxane.

2/ les silicones non volatiles du type polyalkylsiloxane linéaire à groupements terminaux triméthylsilyle, telles que les huiles SILBIONE 70047 et 47 telle que l'huile 47 V 500 000, commercialisées par RHONE POULENC, ou du type polyalkylarylsiloxane comme l'huile SILBIONE 70641V200 commercialise par RHONE POULENC.

3/ les mélanges d'organosiloxanes et de silicones cycliques tels que la Q2 1401 de la Société DOW CORNING, la SF 1214 SILICONE FLUID de la Société GENERAL ELECTRIC.

4/ la résine (diméthyl/triméthylpolysiloxane) vendue sous le nom de DOW CORNING 593 par la Société DOW CORNING.

5/ les polyorganosiloxanes organomodifiés choisis parmi les huiles PDMS à fonction hydroxypropyle de formule (I) telle que définie ci-dessus, dans laquelle $R_1$ désigne méthyle et $R'_1$ un groupement triméthylène ou méthyl-2 triméthylène; les huiles PDMS à groupement acyloxyalkyle de formule (II) telle que définie ci-dessus, dans laquelle $R_2$ et $R'_2$ désignent un radical méthyle, R" désigne un mélange de radicaux $C_{16}H_{33}$ et $C_{18}H_{37}$ et R désigne un triméthylène ou un méthyl-2 triméthylène; les huiles à groupement itaconate de diméthyle, répondant à la formule (III) définie ci-dessus, dans laquelle $R_3$ désigne $CH_3$ et Z' désigne OH.

Les polyorganosiloxanes utilisés conformément à la présente invention sont présents dans la dispersion aqueuse dans une proportion comprise entre 0,5 et 50% en poids et de préférence entre 1 et 30% en poids, par rapport au poids total de la dispersion.

Le copolymère d'acrylate d'ammonium/acrylamide réticulé, utilisé conformément à la présente invention, est plus particulièrement un copolymère acrylate d'ammonium/acrylamide (95/5 en poids) réticulé par un composé à polyinsaturation oléfinique tel que le divinylbenzène, le tétraallyloxyéthane, l'éther diallylique, des éthers polyallylpolyglycéryliques ou les éthers allyliques d'alcools de la série des sucres, tels que l'érythritol, le pentaérytritol, l'arabitol, le mannitol, le sorbitol ou le glucose.

Des copolymères analogues sont décrits et préparés dans le brevet français FR-2 416 723 (HOECHST).

D'autres procédés d'obtention de ce type de copolymère sont décrits également dans les brevets US 2 798 053 et US 2 923 692.

Le copolymère d'acrylate d'ammonium/acrylamide réticulé est présent dans les dispersions aqueuses selon l'invention, dans des proportions comprises entre 0,1 et 10% en poids et de préférence entre 0,1 et 4% en poids par rapport au poids total des dispersions.

Une forme d'utilisation préférentielle de dispersion aqueuse en cosmétique ou dermatologie selon la présente invention, consiste à mettre en oeuvre une dispersion contenant dans un milieu aqueux cosmétiquement ou physiologiquement acceptable :

a) un organopolysiloxane;

b) un copolymère d'acrylate d'ammonium/acrylamide réticulé;

c) un agent émulsionnant non-ionique;

d) une huile minérale.

L'huile minérale utilisée selon la présente invention est un mélange d'hydrocarbures distillant entre 250°C et 300°C, obtenue dans la rectification du pétrole brut. On utilise de préférence l'huile de paraffine. L'huile est présente dans les dispersions utilisées selon l'invention, dans des proportions comprises entre 0,08 et 6,5% en poids et de préférence entre 0,08 et 2,5% en poids par rapport au poids total des dispersions.

Les agents émulsionnants non-ioniques utilisés dans la présente invention sont choisis de préférence parmi les tensio-actifs oléosolubles ayant un rapport hydrophile/lipophile (ou HLB) inférieur à 7.

On utilise de préférence comme agent émulsionnant non-ionique, un mélange de stéarate de sorbitan et d'un dérivé éthoxylé hydrophile.

L'agent émulsionnant est présent dans les dispersions utilisées selon l'invention, dans des proportions comprises entre 0,01 et 1% en poids et de préférence entre 0,01 et 0,6% en poids.

Une forme particulièrement préférée de dispersion aqueuse utilisée selon l'invention contient le copolymère d'acrylate d'ammonium/acrylamide (95/5 en poids) réticulé par un agent de réticulation à polyinsaturation oléfinique, dispersé dans une émulsion eau-dans-huile constituée par 30% en poids dudit polymère, 25% en poids de paraffine, 4% de mélange de stéarate de sorbitan et de dérivé éthoxylé hydrophile, 41% en poids d'eau. Une telle émulsion est commercialisée sous le nom de PAS 5161 par la Société HOECHST.

Cette dispersion aqueuse particulière, utilisée selon l'invention, est de préférence préparée par simple mélange à température ambiante et sous agitation, du polymère organosiloxane avec l'émulsion définie ci-dessus. Le mélange ainsi obtenu peut être directement introduit dans l'eau renfermant d'autres ingrédients choisis en fonction de l'application désirée.

L'émulsion eau-dans-huile renfermant le copolymère acrylate d'ammonium/acrylamide réticulé (95/5 en poids), est de préférence présente dans la dispersion aqueuse dans des proportions telles que la concentration en copolymère soit comprise entre 0,05 et 10% en poids et de préférence entre 0,1 et 5% en poids de matière active de copolymère, par rapport au poids total de la dispersion.

Un autre objet de l'invention est constitué par une composition sous forme de dispersion aqueuse destinée au traitement des cheveux ou de la peau en cosmétique et/ou en dermatologie, caractérisée par le fait que la dispersion aqueuse est telle que définie précédemment.

Les compositions conformes à la présente invention peuvent contenir en plus des adjuvants habituellement utilisés en cosmétique tels que des parfums, des colorants, des conservateurs, des agents séquestrants, des huiles végétales, animales ou synthétiques, des filtres solaires, des agents tensio-actifs anioniques,non ioniques, amphotères ou cationiques, des polymères, des agents de conditionnement, des stabilisateurs de mousse, des propulseurs ou autres adjuvants habituellement utilisés dans les compositions pour les chevaux ou la peau, suivant l'application envisagée.

Les compositions cosmétiques destinées au traitement des cheveux, conformes à l'invention, peuvent être utilisées en particulier comme shampooing, comme produit à rincer, à appliquer avant ou après shampooing, avant ou après coloration ou décoloration, avant ou après permanente ou défrisage ou comme produit coiffant non rincé, tel que dans des lotions de mises en plis ou de brushing.

Les compositions cosmétiques conformes à la présente invention destinées au traitement et aux soins de la peau, peuvent être sous forme de produit pour le bain ou la douche, de produit bronzant, de produit pour le rasage, de lotion parfumée, de crème ou de lait pour le soin de la peau ou des compositions antisolaires.

Les compositions conformes à la présente invention peuvent être appliquées en dermatologie. Elles contiennent en une quantité efficace une substance active sur le plan dermatologique, telle que par exemple la vitamine A, les caroténoïdes, les protéines, les pigments naturels, des rétinoïdes, des dépigmentants, des substances antiséborrhéiques ou antiacnéïques, des anti-inflammatoires.

Les compositions cosmétiques ou dermatologiques selon la présente invention présentent un pH compris entre 3 et 10 et de préférence entre 5 et 7. Ce pH peut être ajusté par des agents alcalinisants ou acidifiants habituellement utilisés en cosmétique et en dermatologie.

Un procédé de traitement cosmétique des cheveux selon l'invneiton consiste à appliquer les compositions telles que définies ci-dessus sur les cheveau suivant l'usage envisagé (shampooing, traitement à rincer, traitement de coiffage sans rinçage), sans qu'il soit nécessaire d'observer un temps de pose.

Un procédé de traitement cosmétique de la peau selon l'invention consiste à appliquer sur celle-ci une composition telle que définie ci-dessus, selon l'usage envisagé (bain, douche, produits bronzants, produits pour le rasage, lotions parfumées, crèmes ou laits de soin).

Les exemples qui suivent sont destinés à illustrer la présente invention, sans pour autant présenter un caractère limitatif.

EXEMPLE DE REFERENCE 1

Préparation d'un silane de formule (IIIC), dans laquelle :

$R_3$ désigne $CH_3$

a désigne 1

Z est un mélange de radicaux :

$$-CH_2\text{-}CH(COOCH_3)\text{-}CH_2COOCH_3$$

et

$$-C(CH_3)(COOCH_3)\text{-}CH_2COOCH_3$$

Dans un réacteur tricol de 2 litres, muni d'un réfrigérant, d'un agitateur et d'une ampoule de coulée, on charge 907 g, soit 5,74 moles d'itaconate de méthyle et 129 mg d'acide chloroplatinique ($H_2PtCl_6$).

On porte la température à 116°C, puis on coule pendant 65 minutes 792,5 g (6,89 moles) de $CH_3HSiCl_2$, soit un excès molaire de 20% par rapport à l'itaconate. La réaction étant exothermique, la température se maintient aux environs de 120°C sans addition supplémentaire de calories. En fin de coulée, la température est de 112°C. On maintient le mélange réactionnel au reflux durant 1 heure 50 minutes, puis on distille l'excès de $CH_3HSiCl_2$ et on obtient 1274 g d'un adduct liquide dont le point d'ébullition est de 80°C sous 0,13 KPa. Le rendement pondéral en adduct est de 71%.

L'analyse RMN de l'adduct indique qu'il présente environ 60% molaire de radicaux
$-(CH_2)-CH(COOCH_3)-CH_2-COOCH_3$
et 40% molaire de motif
$-C(CH_3)(COOCH_3)-CH_2-COOCH_3$.

EXEMPLE DE REFERENCE 2

Préparation d'un composé de formule (III), dans laquelle :
$R_3$ désigne $CH_3$;
Z′ désigne OH;
Z est un mélange de radicaux :

$$-CH_2-CH(COOCH_3)-CH_2-COOCH_3$$

et

$$-C(CH_3)(COOCH_3)-CH_2-COOCH_3;$$

r représente une valeur statistique moyenne égale à 40;
s représente une valeur statistique moyenne égale à 2.

Dans un réacteur tricol de 10 litres, on charge 2800 g d'eau sur laquelle on coule pendant une heure 903 g (7 moles) de $(CH_3)_2SiCl_2$ et 98 g (0,35 mole) de l'adduct obtenu à l'exemple de référence 1. Durant la coulée, la température s'élève graduellement de 25 à 65°C. Après la coulée, on maintient le mélange réactionnel sous agitation pendant 30 minutes et on décante les eaux acides. On ajoute 350 ml d'éther isopropylique, on effectue trois lavages et on concentre la solution éthérée suivant on premier palier jusqu'à 100°C à pression atmosphérique, puis jusqu'à 80°C sous un vide de 2,5 KPa.

On obtient alors 456 g d'huile limpide et incolore, ayant les caractéristiques suivantes :

| | |
|---|---|
| Viscosité à 25°C | 20 mPa.s |
| % (pondéral) d'hydroxyle | 1% |
| % (pondéral) de fonction ester | 4,8% |
| Rendement pondéral en huile | 77% |

EXEMPLE DE REFERENCE 3

Préparation d'un composé de formule moyenne (II), dans laquelle :
$R_2 = R'_2 = CH_3$, $R = -(CH_2)_3-$
$p = 7,9$
$q = 1,4$
$r = 9,3$
$R'' =$ mélange de radicaux $C_{16}H_{33}$ et $C_{18}H_{37}$.

Ce composé comporte en moyenne environ deux motifs

$$CH_3\underset{\substack{|\\O_{2/2}}}{Si}OH$$

Dans un ballon tricol de 3 litres, équipé d'un agitateur, muni d'un dispositif de chauffage, d'un réfrigérant ascendant et balayé par un courant d'azote par bullage dans le milieu réactionnel, on charge 800 g d'une huile polydiméthylsiloxane à fonction γ-hydroxypropylée (obtenue par réaction d''hydrosilylation d'alcool allylique par une huile polydiméthylpolyméthylhydrogéno-siloxane en présence d'un catalyseur au platine) de structure moyenne, déterminée par analyse $RMN^{29}Si$ répondant à la formule moyenne (I), dans laquelle :
$R_1 = CH_3$
$R'_1 = -(CH_2)_3-$
$p = 6,8$
$q = 6,8$
et comportant en moyenne un motif

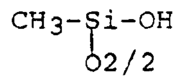

titrant 459,5 meq/100 g en fonction alcool propylique, 1207 g d'une coupe d'esters gras R″$CO_2$Me (R″ ayant la composition :

| | |
|---|---|
| $C_{14}H_{29}$ | 0,5 |
| $C_{16}H_{33}$ | 37,0 |
| $C_{18}H_{37}$ | 61,0 |
| $C_{20}H_{41}$ | 1,5 |
| | 100% poids |

Le milieu est porté par chauffage à 120°C. Lorsque la température est atteinte, on ajoute 6,02 g d'acide paratoluènesulfonique monohydraté ($CH_3C_6H_4SO_3H$, $H_2O$).

On effectue la réaction sous agitation pendant 17 heures. En fin de réaction, on ajoute dans le milieu réactionnel 500 ml d'hexane, l'élimination du catalyseur acide est alors effectuée par lavage, neutralisation avec $NaHCO_3$ aqueux.

La phase hexanique est séchée sur $Na_2SO_4$ et filtrée. 1822,6 g d'une huile limpide de couleur ambrée où 85% des fonctions alcool sont transformées en fonction ester gras, sont obtenus après élimination de l'hexane par distrillation à 110°C sous 1,33 KPa pendant 3 heures. L'huile prend l'aspect d'une cire lors du refroidissement à température ambiante.

L'analyse RMN$^{29}$Si indique la structure mentionnée ci-dessus.

La teneur résiduelle en fonction alcool propylique est de 37 meq/100 g et en esters gras méthyliques $C_{16}$+$C_{18}$ de 20% en poids.

EXEMPLE 1

On prépare une composition après-shampooing à ne pas rincer, qui se présente sous forme de gel fluide, dont la formulation est la suivante :

- Emulsion de copolymère acrylate d'ammonium/acrylamide réticulé, vendue sous la dénomination PAS 5161 par la Société HOECHST 0,50 g MA en copolymère
- Polyorganosiloxane hydroxypropylé de formule :

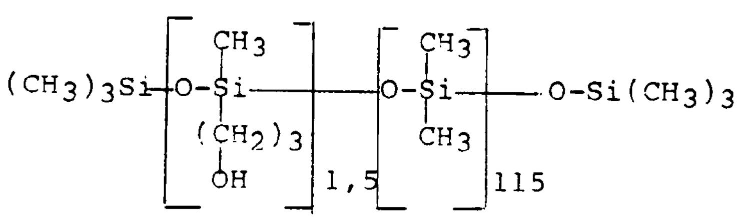 10,0 g

($\bar{M}$n ~ 9.000)

- N-(1,3 bis-hydroxyméthyl)-2,5
- dioxo 4-imidazolidinylurée vendu par la Société SUTTON sous la dénomination GERMALL II 0,1 g
- Eau qsp 100,0 g
- pH spontané : 6,25

On applique cette composition sur des cheveux lavés et essorés. On ne rince pas et l'on procède au séchage. Les cheveux mouillés et séchés se démêlent remarquablement bien. Les cheveux séchés sont doux et brillants.

EXEMPLE 2

On prépare une crème de soin pour cheveux abîmés, à ne pas rincer, dont la composition est la suivante :

- Emulsion de copolymère acrylate d'ammonium/acrylamide réticulé, vendue sous la dénomination PAS 5161 par la Société HOECHST 1,0 g MA en copolymère
- Polyorganosiloxane à fonction acyloxypropyle de formule :

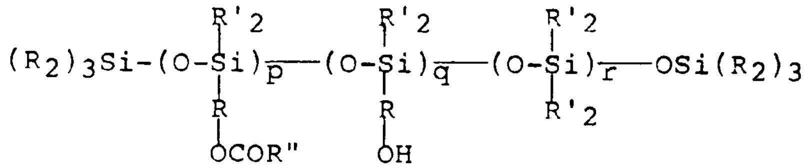 5,0 g

dans laquelle :

. $R_2 = R'_2, = CH_3$, $R = (CH_2)_3$
. p = 7,9
. q = 1,4
. r = 9,3
. R" = mélange de radicaux $C_{16}H_{33}$ et $C_{18}H_{37}$.

Ce composé comporte en moyenne environ deux motifs

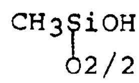

préparé selon l'exemple de référence 3

- N-(1,3-bis-hydroxyméthyl)-2,5
- dioxo 4-imidazolinidilurée vendu par la Société SUTTON sous la dénomination GERMALL II 0,1 g
- Eau qsp 100,0 g
- pH spontané : 6

On applique cette crème onctueuse sur des cheveux lavés et essorés. Elle améliore le démêlage des cheveux mouillés et séchés, et rend les cheveux séchés doux et lisses.

EXEMPLE 3

On prépare un lait traitant, à ne pas rincer, pouvant être appliqué comme après-shampooing sur des cheveux propres et mouillés ou comme soin de finition sur des cheveux séchés, dont la composition est la suivante :

- Emulsion de copolymère acrylate d'ammonium/acrylamide réticulé, vendue sous la dénomination PAS 5161 par la Société HOECHST 0,25 g MA en copolymère
- Polydiméthyldiphénylsiloxane vendu par la Société RHONE POULENC sous la dénomination huile SILBIONE 70641 V 200 0,5 g
- N-(1,3 bis-hydroxyméthyl)-2,5
- dioxo 4-imidazolidinylurée vendu par la Société SUTTON sous la dénomination GERMALL II 0,1 g
- Eau qsp 100,0 g
- pH spontané : 6,6

Ce lait appliqué sur cheveux lavés et essorés facilite le démêlage des cheveux mouillés, apporte de la légèreté aux cheveux mouillés et séchés.

Lorsqu'il est appliqué en soin de finition, il facilite le coiffage et apporte du gonflant à la coiffure.

EXEMPLE 4

On prépare une crème après-shampooing que l'on rince et dont la composition est la suivante :

- Emulsion de copolymère acrylate d'ammonium/acrylamide réticulé, vendue sous la dénomination PAS 5161 par la Société HOECHST 2,0 g Ma en copolymère
- Polyorganosiloxane à fonction itaconate de diméthyle, de formule :

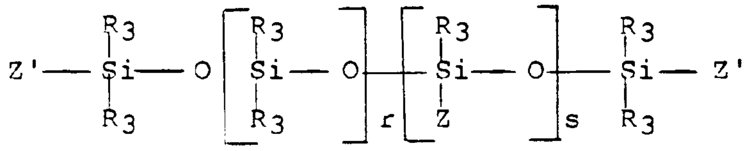 15,0 g

dans laquelle :

. $R_3$ désigne $CH_3$

. Z' désigne OH

. Z est un mélange de radicaux :

$-CH_2-CH(COOCH_3)-CH_2-COOCH_3$

$-C(CH_3)(COOCH_3)-CH_2-COOCH_3$

. r représente une valeur statistique moyenne égale à 40

. s représente une valeur statistique moyenne égale à 2

préparé selon l'exemple de référence 2

- -N-(1,3 bis-hydroxyméthyl)-2,5
- dioxo 4-imidazolidinylurée vendu par la Société SUTTON sous la dénomination GERMALL II 0,1 g
- Eau qsp 100,0 g
- pH spontané : 5,6

Cette crème s'applique sur cheveux lavés et essorés. Après quelques minutes de pose, on rince à l'eau. Les cheveux sont faciles à démêler. Ils sont gonflants et légers; les cheveux séchés sont brillants, doux, lisses et faciles à coiffer.

## EXEMPLE 5

On prépare une crème que l'on applique en après-shampooing à rincer, de composition suivante :

- Emulsion de copolymère acrylate d'ammonium/acrylamide réticulé, vendue sous la dénomination PAS 5161 par la Société HOECHST 1,5 g MA en copolymère
- PDMS hydroxylé en bout de chaîne associé à un PDMS cyclique, vendu par la Société DOW CORNING sous la dénomination Q2 1401 5,0 g
- Chlorure de distéaryldiméthylammonium vendu par la Société HOECHST sous la dénomination GENAMIN DSAC 2,5 g
- N-(1,3 bis-hydroxyméthyl)-2,5
- dioxo 4-imidazolidinylurée vendu par la Société SUTTON sous la dénomination GERMALL II 0,1 g
- Eau qsp 100,0 g
- pH spontané : 5,7

Après le shampooing, on applique cette crème sur les cheveux essorés, on laisse poser quelques minutes, puis on rince à l'eau. Les cheveux mouillés sont faciles à démêler et deviennent souples et légers. Les cheveux séchés sont gonflants, brillants et doux.

## EXEMPLE 6

On prépare une crème de coiffage qui s'applique sur cheveux séchés en soin de finition, dont la composition est la suivante :

- Emulsion de copolymère acrylate d'ammonium/acrylamide réticulé, vendu sous la dénomination PAS 5161 par la Société HOECHST 0,3 g MA en copolymère
- PDMS hydroxylé en bout de chaîne associé à un PDMS cyclique, vendu par la Société DOW CORNING sous la dénomination Q2 1401 20,0 g
- N-(1,3 bis-hydroxyméthyl)-2,5
- dioxo 4-imidazolidinylurée vendu par la Société SUTTON sous la dénomination GERMALL II 0,1 g
- Eau qsp 100,0 g
- pH spontané : 6,5

Cette crème se répartit facilement sur les cheveux; elle leur apporte un démêlage aisé, de la brillance, du gonflant et de la discipline.

EXEMPLE 7

On prépare un shampooing de composition suivante :

- Emulsion de copolymère acrylate d'ammonium/acrylamide réticulé, vendue sous la dénomination PAS 5161 par la Société HOECHST 0,8 g MA en copolymère
- Tensio-actif non ionique poly hydroxypropyléther) préparé par condensation, en catalyse alcaline, de 3,5 moles de glycidol sur un mélange d'α-diols en CII-14 selon le procédé décrit dans le brevet français n° 2091516 10,0 g
- Polyorganosiloxane hydroxypropylé de formule :

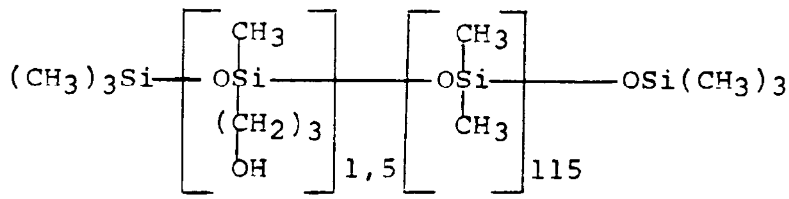

2,0 g

(Mn ∼ 9.000)

- N-(1,3 bis-hydroxyméthyl-2,5
- dioxo 4-imidazolidinylurée vendu par la Société SUTTON sous la dénomination GERMALL II 0,1 g
- Eau qsp 100,0 g
- pH spontané : 5,9

Ce shampooing, sous l'aspect d'un gel fluide, opaque, laisse les cheveux mouillés légers et souples, et les cheveux séchés doux et faciles à coiffer.

EXEMPLE 8

On prépare un gel fluide hydratant pour la peau, de composition suivante :

- Emulsion de copolymère acrylate d'ammonium/acrylamide réticulé, vendue sous la dénomination PAS 5161 par la Société HOECHST 0,5 g MA en copolymère
- PDMS hydroxylé en bout de chaîne associé à un PDMS cyclique, vendu par la Société DOW CORNING sous la dénomination Q2 1401 15,0 g
- Glycérine 5,0 g
- N-(1,3 bis-hydroxyméthyl)-2,5
- dioxo 4-imidazolidinylurée vendu par la Société SUTTON sous la dénomination GERMALL II 0,1 g
- Parfum qs
- Eau qsp 100,0 g
- pH spontané : 6,2

Ce gel apporte beaucoup de douceur et de souplesse à la peau.

EXEMPLE 9

On prépare une composition de coiffage de composition suivante :

- Emulsion de copolymère acrylate d'ammonium/acrylamide réticulé, vendue sous la dénomination PAS 5161 par la Société HOECHST 0,3 g en copolymère
- PDMS hydroxylé en bout de chaîne à un PDMS cyclique vendu par la Société DOW CORNING sous la dénomination Q2 1401 20,0 g
- Polyéthyloxazoline de PM 50 000 vendue sous la dénomination PEOX par la Société DOW CHEMICAL 3,5 g
- Conservateur, colorant, parfum qs
- Eau qsp 100,0 g
- pH spontané : 6

On applique ce gel sur cheveux propres et séchés. La composition améliore le coiffage et apporte de la tenue et de la brillance.

EXEMPLE 10

On prépare une mousse de coiffage qui s'applique sur cheveux séchés en soin de finition dont la composition est la suivante :

- Emulsion de copolymère acrylate d'ammonium/acrylamide réticulé, vendue sous la dénomination PAS 5161 par la Société HOECHST 0,2 g MA en copolymère
- PDMS hydroxylé en bout de chaîne associé à un PDMS cyclique, vendu par la Société DOW CORNING sous la dénomination Q2 1401 13,0 g
- Alcool éthylique 10,0 g
- Alcool polyvinylique vendu sous la dénomination MOWIOL 4088 par la Société HOECHST 0,3 g MA
- Parfum, conservateur qs
- Eau qsp 100,0 g
- Triéthanolamine qs pH = 6,5

CONDITIONNEMENT AEROSOL :

| | |
|---|---|
| Composition ci-dessus | 90,0 g |

PROPULSEUR :

| | |
|---|---|
| Mélange ternaire de n-butane, isobutane > 55%, propane vendu sous la dénomination AEROGAZ 3,2 N par la Société ELF AQUITAINE | 10,0 g |
| TOTAL | 100,0 g |

Cette mousse peut également être appliquée sur des cheveux lavés et essorés avant le séchage final. Les cheveux sont brillants, gonflants, se démêlent aisément et sont très doux.

EXEMPLE 11

COMPOSITION SOLAIRE

- Emulsion de copolymère acrylate d'ammonium/acrylamide réticulé, vendue sous la dénomination PAS 5161 par la Société HOECHST 0,4 g MA en copolymère
- PDMS hydroxylé en bout de chaîne associé à un PDMS cyclique, vendu sous la dénomination Q2 1401 par la Société DOW CORNING 13,0 g
- 2-hydroxy 4-méthoxybenzophénone vendu sous la dénomination UVINUL M40 par la Société BASF 2,0 g
- p-méthoxycinnamate de 2-éthylhexyle vendu dous la dénomination PARSOL MCX par la Société GIVAUDAN 5,0 g
- Conservateur qs
- Eau qsp 100,0 g

EXEMPLE 12

COMPOSITION APRES-SOLEIL

- Emulsion de copolymère acrylate d'ammonium/acrylamide réticulé, vendue sous la dénomination PAS

5161 par la Société HOECHST 0,4 g MA en copolymère
- PDMS hydroxylé en bout de chaîne associé à un PDMS cyclique, vendu sous la dénomination Q2 1401 par la Société DOW CORNING 13,0 g
- Glycérine 3,0 g
- Sorbitol à 70% MA 1,4 g MA
- Conservateur qs
- Eau qsp 100,0 g

EXEMPLE 13

On prépare une crème anti-inflammatoire de composition suivante :
- Emulsion de copolymère acrylate d'ammonium/acrylamide réticulé vendue sous la dénomination PAS 5161 par la Société HOECHST 1,5 g MA en copolymère
- Sel d'ammonium d'acide glycyrrhizinique 0,5 g
- Polyméthylphénylsiloxane vendu sous la dénomination ABIL AV 1000 par la Société GOLDSCHMIDT 5,0 g
- Parfum, conservateur, colorant qs
- pH spontané : 5,3
- Eau qsp 100,0 g

EXEMPLE 14

On prépare une crème anti-acnéique de composition suivante :
- Emulsion de copolymère acrylate d'ammonium/acrylamide réticulé, vendue sous la dénomonation PAS 5161 par la Société HOECHST 3,0 g MA en copolymère
- Acide retinoïque 0,1 g
- PDMS vendu sous la dénomination 7004V300 par la Société RHONE POULENC 20,0 g
- Parfum, conservateru, colorant qs
- pH spontané : 5,4
- Eau qsp 100,0 g

Dans les exemples ci-dessus, le poids moléculaire du polymère acrylate d'ammonium/acrylamide avant réticulation, est de l'ordre de $5.10^6$.

## Revendications

### Revendications pour les Etats contractants suivants : AT, BE, CH, DE, DK, FR, GB, IT, LI, NL, SE

**1.** Utilisation en cosmétique, pour le traitement des cheveux ou de la peau et/ou en dermatologie, d'une dispersion aqueuse, caractérisée par le fait que ladite dispersion contient au moins dans un milieu aqueux cosmétiquement ou physiologiquement acceptable, un organopolysiloxane et un copolymère d'acrylate d'ammonium/acrylamide réticulé.

**2.** Utilisation selon la revendication 1, caractérisée par le fait que l'organosiloxane est une silicone volatile ayant un point d'ébullition compris entre 60°C et 260°C, choisie parmi :

(i) les silicones cycliques de 3 à 7 atomes de silicium et de préférence 4 à 5 ou les cyclocopolymères du type diméthylsiloxane/méthylalkylsiloxane, de formule :

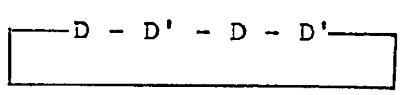

dans laquelle :

D représente :

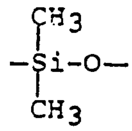

et D' représente :

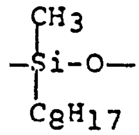

(ii) les silicones linéaires, ayant 2 à 9 atomes de silicium et possédant une viscosité inférieure ou égale à $5.10^{-6}$ $m^2/s$ à 25°C.

**3.** Utilisation selon l'une quelconque des revendications 1 à 2, caractérisée par le fait que l'organopolysiloxane est une silicone non volatile choisie parmi les polyalkylsiloxanes, les polyarylsiloxanes, les polyalkylarylsiloxanes, les copolymères polyéthersiloxanes, modifiés ou non, les gommes et résines de silicone, les polysiloxanes organomodifiés, ainsi que leurs mélanges.

**4.** Utilisation selon la revendication 3, caractérisée par le fait que l'organopolysiloxane est choisi parmi :
A/ les polyalkyl($C_1$-$C_{20}$)siloxanes, les polydiméthylsiloxanes linéaires à groupements terminaux triméthylsilyle, de viscosité $5.10^{-6}$ à $2,5.10^{-1}$ $m^2/s$ à 25°C et de préférence $10^{-5}$ à 1 $m^2/s$ et les polydiméthylsiloxanes linéaires, à groupements terminaux trihydroxysilyle;
B/ les polydiméthylphénylsiloxanes, les polydiméthyldiphénylsiloxanes linéaires et/ou ramifiés, de viscosité $10^{-5}$ à $5.10^{-2}$ $m^2/s$ à 25°C;
C/ les copolymères d'oxyde d'éthylène et/ou de propylène avec un diorganosiloxane;
D/ les gommes de masse moléculaire comprise entre 200.000 et 1.000.000, utilisées seules ou sous forme de mélange dans un solvant, choisies dans le groupe constitué par les copolymères suivants :

- poly/(diméthylsiloxane)/(méthylvinylsiloxane)/
- poly/(diméthyl siloxane)/(diphénylsiloxane)/
- poly/(diméthyl siloxane)/(phénylméthylsiloxane)/
- poly/(diméthylsiloxane)/(diphénylsiloxane)/ (méthylvinylsiloxane)/;

et les mélanges suivants :

- les mélanges formés à partir d'un polydiméthylsiloxane hydroxylé en bout de chaîne et les polydiméthylsiloxanes cycliques;
- les mélanges formés à partir d'une gomme polydiméthylsiloxane et d'une silicone cyclique;
- les mélanges de deux polydiméthylsiloxanes de viscosités différentes;
- les mélanges d'un polydiméthylsiloxane à chaîne latérale polyoxyéthylénée ou polyoxypropylénée et d'un polydiméthylsiloxane cyclique;

E/ les résines d'organopolysiloxanes constituées de systèmes siloxaniques réticulés, renfermant les unités $R_2SiO_{2/2}$, $RSiO_{3/2}$, $SiO_{4/2}$, dans lesquelles R représente un groupement hydrocarboné possédant 1 à 6 atomes de carbone ou un groupement phényle.

**5.** Utilisation selon la revendication 3, caractérisée par le fait que l'organopolysiloxane comporte dans sa structure générale un ou plusieurs groupement(s) organofonctionnel(s) directement fixé(s) sur la chaîne siloxanique ou fixé(s) par l'intermédiaire d'un radical hydrocarboné, et qu'il est choisi parmi les polyorganosiloxanes comportant :
a/ des groupements aminés substitués ou non;
b/ des groupements thiol;
c/ des groupements carboxylate;
d/ des groupements hydroxyalkyle,
et répondant à la formule suivante :

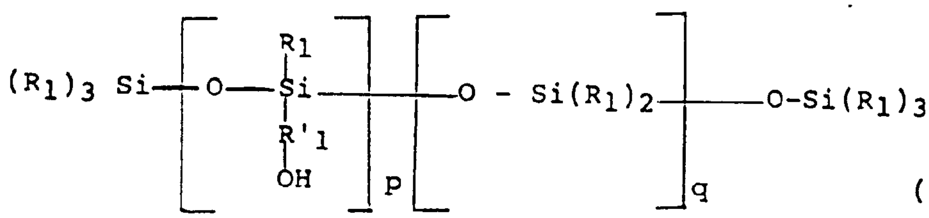

(I)

dans laquelle :

- les radicaux $R_1$, identiques ou différents, sont choisis parmi les radicaux méthyle et phényle, au moins 60% en mole des radicaux $R_1$ étant méthyle;
- le radical $R'_1$ est un chaînon alkylène divalent hydrocarboné en $C_2$-$C_{18}$;
- p est compris entre 1 et 30 inclus;
- q est compris entre 1 et 150 inclus;

e/ des groupements alcoxylés;

f/ des groupements acyloxyalkyle répondant à la formule suivante :

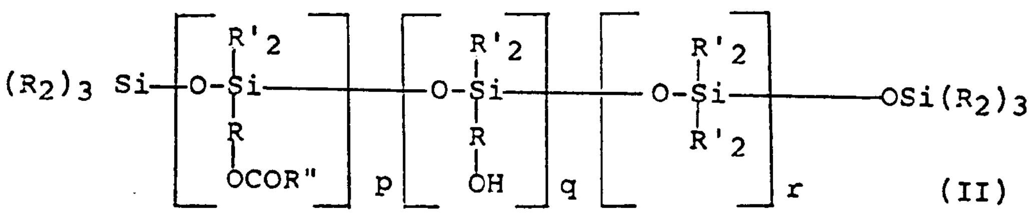

(II)

dans laquelle :

- $R_2$ désigne méthyle,phényl, $COOR_2$", hydroxyle, un seul des $R_2$ par atome de silicium peut être OH;
- $R'_2$ désigne méthyle, phényle, 60% molaire au moins de l'ensemble des radicaux $R_2$ et $R'_2$ est méthyle;
- R" désigne alcoyle ou alcényle en $C_8$-$C_{20}$;
- R désigne un alkylène divalent hydrocarboné, linéaire ou ramifié, en $C_2$-$C_{18}$;
- r est compris entre 1 et 120 inclus;
- p est compris entre 1 et 30;
- q est 0 ou inférieur à 0,5 p, p+q étant compris entre 1 et 30;

les polymères de formule (II) pouvant contenir des groupements

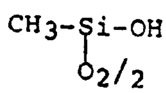

dans des proportions ne dépassant pas 15% de la somme p+q+r;

g/ des groupements diester, en particulier itaconate de diméthyle, répondant à la formule suivante :

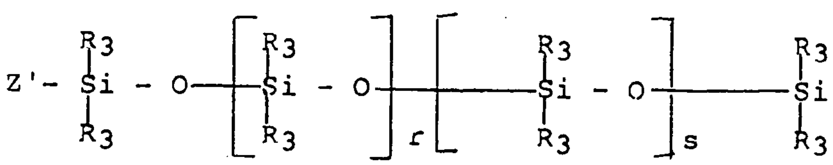

(III)

dans laquelle :

- les radicaux $R_3$, identiques ou différents, désignent un alkyle en $C_1$-$C_{20}$, vinyle, phényle, trifluoro-3,3,3 propyle ou hydroxyle, sous réserve qu'un seul radical $R_3$ peut désigner OH par atome de silicium;
- les symboles Z', identiques ou différents, désignent $R_3$ ou Z;
- Z est choisi parmi les groupes :

$CH_2$-CH(COOR'$_3$)-$CH_2$-COOR'$_3$
C($CH_3$)(COOR'$_3$)-$CH_2$-COOR'$_3$

- R'$_3$ désigne un radical hydrocarboné saturé, monovalent en $C_1$-$C_{12}$, un alcoxyalkyle, monovalent en $C_2$-$C_{12}$, un aryle, aralkyle ou alkylaryle en $C_6$-$C_{12}$;
- r est compris entre 0 et 500 inclus;
- s est compris entre 0 et 50 inclus;
- si s est égal à 0, un des symboles Z' est Z;

h/ des groupements ammonium quaternaire;
i/ des groupements amphotères ou bétaïniques;
j/ des groupements anioniques de type alkylcarboxylique 2-hydroxyalkylsulfonate ou 2-hydroxyalkylthiosulfate.

6. Utilisation selon l'une quelconque des revendications 1 à 5, caractérisée par le fait que l'organopolysiloxane est choisi parmi l'octaméthylcyclotétrasiloxane; le décaméthylcyclopentasiloxane; les polyalkylsiloxanes linéaires à groupements terminaux triméthylsilyle; les mélanges d'organosiloxanes et de silicones cycliques, la résine diméthyl/triméthylsiloxane; les polymères de formule (I), dans laquelle $R_1$ désigne méthyle et R'$_1$ un groupement triméthylène ou méthyl-2 triméthylène;les polymères de formule (II), dans laquelle $R_2$ et R'$_2$ désignent méthyle, R" un mélange de radicaux $C_{16}H_{33}$ et $C_{18}H_{37}$ et R désigne triméthylène ou méthyl-2 triméthylène; les polymères de formule (III), dans laquelle $R_3$ désigne $CH_3$ et Z' désigne OH.

7. Utilisation selon l'une quelconque des revendications 1 à 6, caractérisée par le fait que la dispersion aqueuse contient un copolymère d'acrylate d'ammonium/acrylamide (95/5 en poids), réticulé par un agent réticulant à polyinsaturation oléfinique pris dans le groupe constitué par le divinylbenzène, le tétraallyloxyéthane, l'éther diallylique, des éthers polyallylpolyglycéryliques et les éthers allyliques d'alcools de la série des sucres.

8. Utilisation selon l'une quelconque des revendications 1 à 7, caractérisée par le fait que l'organopolysiloxane est présent dans la dispersion dans des proportions comprises entre 0,5 et 50% en poids et de préférence entre 1 et 30% en poids et le copolymère acrylate d'ammonium/acrylamide est présent dans des proportions comprises entre 0,1 et 10% en poids et de préférence entre 0,1 et 4% en poids par rapport au poids total de la dispersion.

9. Utilisation selon l'une quelconque des revendications 1 à 8, caractérisée par le fait que la dispersion contient en plus au moins un agent émulsionnant non-ionique et une huile minérale.

10. Utilisation selon la revendication 9, caractérisée par le fait que l'agent émulsionnant non-ionique est choisi parmi les tensio-actifs oléosolubles dont le rapport hydrophile/lipophile est inférieur à 7.

11. Utilisation selon la revendication 9 ou 10, caractérisée par le fait que l'agent émulsionnant est constitué par un mélange de stéarate de sorbitan et d'un dérivé éthoxylé hydrophile.

12. Utilisation selon la revendication 9, caractérisée par le fait que l'huile minérale est l'huile de paraffine.

13. Utilisation selon l'une quelconque des revendications 9 à 12, caractérisée par le fait que l'agent émulsionnant non-ionique est présent dans des proportions comprises entre 0,01 et 10% en poids, et de préférence entre 0,01 et 0,6% en poids et l'huile minérale est présente dans des proportions comprises entre 0,08 et 6,5% en poids, et de préférence entre 0,08 et 2,5% en poids, les poids étant définis par rapport au poids total de la dispersion.

14. Utilisation selon l'une quelconque des revendications 1 à 13, caractérisée par le fait que le copolymère acrylate d'ammonium/acrylamide réticulé (95/5 en poids) est dispersé à raison de 30% en poids dans une émulsion eau-dans-huile constituée de 25% en poids de paraffine, 4% en poids de mélange de stéarate de sorbitan et de dérivé éthoxylé hydrophile et de 41% en poids d'eau.

15. Utilisation de la dispersion aqueuse selon l'une des revendications 1 à 14, caractérisée par le fait que l'émulsion eau-dans-huile contenant le copolymère acrylate d'ammonium/acrylamide réticulé (95/5 en poids) est présente dans la dispersion dans des proportions telles que la concentration en copolymère soit comprise entre 0,05 et 10% en poids et de préférence entre 0,1 et 5% en poids de matière active en copolymère par rapport au poids total de la dispersion.

**16.** Composition cosmétique sous forme de dispersion aqueuse destinée au traitement des cheveux ou de la peau, caractérisée par le fait que la dispersion aqueuse est telle que définie dans l'une quelconque des revendications 1 à 15.

**17.** Composition selon la revendication 16, caractérisée par le fait qu'elle contient en plus des adjuvants habituellement utilisés en cosmétique, choisis parmi les parfums, les colorants, les conservateurs, les agents tensio-actifs anioniques, non-ioniques, amphotères ou cationiques, les séquestrants, les stabilisateurs de mousse, des polymères, des filtres solaires, des propulseurs, des substances actives sur le plan cosmétique.

**18.** Composition selon l'une des revendications 16 ou 17, caractérisée par le fait qu'elle présente un pH compris entre 3 et 10 et de préférence entre 5 et 7.

**19.** Composition selon l'une quelconque des revendications 16 à 18, destinée au traitement des cheveux, caractérisée par le fait qu'elle se présente sous forme de shampooing, de produit à rincer, à appliquer avant ou après shampooing, avant ou après coloration ou décoloration, avant ou après permanente ou défrisage, ou comme produits de coiffage non rincés.

**20.** Composition selon l'une quelconque des revendications 16 à 18, destinée au traitement de la peau, caractérisée par le fait qu'elle se présente comme produit de bain ou de douche, de produit bronzant, de composition anti-solaire, de produit de rasage, de crème ou de lait pour le soin ou de lotion parfumée.

**21.** Procédé de traitement cosmétique des cheveux, caractérisé par le fait que l'on applique sur ceux-ci au moins une composition telle que définie dans la revendication 19.

**22.** Procédé de traitement cosmétique de la peau, caractérisé par le fait que l'on applique sur celle-ci une composition telle que définie dans la revendication 20.

**23.** Composition dermatologique sous forme de dispersion aqueuse, caractérisée par le fait que la dispersion aqueuse est telle que définie dans l'une quelconque des revendications 1 à 15 et qu'elle contient au moins une substance active sur le plan dermatologique dans une quantité efficace.

**Revendications pour l'Etat contractant suivant : GR**

**1.** Utilisation en cosmétique, pour le traitement des cheveux ou de la peau d'une dispersion aqueuse, caractérisée par le fait que ladite dispersion contient au moins dans un milieu aqueux cosmétiquement ou physiologiquement acceptable, un organopolysiloxane et un copolymère d'acrylate d'ammonium/acrylamide réticulé.

**2.** Utilisation selon la revendication 1, caractérisée par le fait que l'organosiloxane est une silicone volatile ayant un point d'ébullition compris entre 60°C et 260°C, choisie parmi :
(i) les silicones cycliques de 3 à 7 atomes de silicium et de préférence 4 à 5 ou les cyclocopolymères de type diméthylsiloxane/méthylalkylsiloxane, de formule :

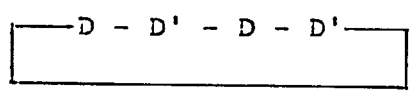

dans laquelle :
D représente :

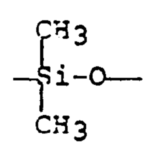

et D' représente :

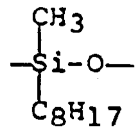

(ii) les silicones linéaires, ayant 2 à 9 atomes de silicium et possédant une viscosité inférieure ou égale à $5.10^{-6}$ $m^2/s$ à 25°C.

**3.** Utilisation selon l'une quelconque des revendications 1 à 2, caractérisée par le fait que l'organopolysiloxane est une silicone non volatile choisie parmi les polyalkylsiloxanes, les polyarylsiloxanes, les polyalkylarylsiloxanes, les copolymères polyéthersiloxanes, modifiés ou non, les gommes et résines de silicone, les polysiloxanes organomodifiés, ainsi que leurs mélanges.

**4.** Utilisation selon la revendication 3, caractérisée par le fait que l'organopolysiloxane est choisi parmi :

A/ les polyalkyl($C_1$-$C_{20}$)siloxanes, les polydiméthylsiloxanes linéaires à groupements terminaux triméthylsilyle, de viscosité $5.10^{-6}$ à $2,5.10^{-1}$ $m^2/s$ à 25°C et de préférence $10^{-5}$ à 1 $m^2/s$ et les polydiméthylsiloxanes linéaires, à groupements terminaux trihydroxysilyle;

B/ les polydiméthylphénylsiloxanes, les polydiméthyldiphénylsiloxanes linéaires et/ou ramifiés, de viscosité $10^{-5}$à $5.10^{-2}$ $m^2/s$ à 25°C;

C/ les copolymères d'oxyde d'éthylène et/ou de propylène avec un diorganosiloxane;

D/ les gommes de masse moléculaire comprise entre 200.000 et 1.000.000, utilisées seules ou sous forme de mélange dans un solvant, choisies dans le groupe constitué par les copolymères suivants :

- poly/(diméthyl siloxane)/(méthylvinylsiloxane)/
- poly/(diméthyl siloxane)/(diphénylsiloxane)/
- poly/(diméthyl siloxane)/(phénylméthylsiloxane)/
- poly/(diméthylsiloxane)/(diphénylsiloxane)/ (méthylvinylsiloxane)/;

et les mélanges suivants :

- les mélanges formés à partir d'un polydiméthylsiloxane hydroxylé en bout de chaîne et les polydiméthylsiloxanes cycliques;
- les mélanges formés à partir d'une gomme polydiméthylsiloxane et d'une silicone cyclique;
- les mélanges de deux polydiméthylsiloxanes de viscosités différentes;
- les mélanges d'un polydiméthylsiloxane à chaîne latérale polyoxyéthylénée ou polyoxypropylénée et d'un polydiméthylsiloxane cyclique;

E/ les résines d'organopolysiloxanes constituées de systèmes siloxaniques réticulés, renfermant les unités $R_2SiO_{2/2}$, $RSiO_{3/2}$, $SiO_{4/2}$, dans lesquelles R représente un groupement hydrocarboné possédant 1 à 6 atomes de carbone ou un groupement phényle.

**5.** Utilisation selon la revendication 3, caractérisée par le fait que l'organopolysiloxane comporte dans sa structure générale un ou plusieurs groupement(s) organofonctionnel(s) directement fixé(s) sur la chaîne siloxanique ou fixé(s) par l'intermédiaire d'un radical hydrocarboné, et qu'il est choisi parmi les polyorganosiloxanes comportant :

a/ des groupements aminés substitués ou non;
b/ des groupements thiol;
c/ des groupements carboxylate;
d/ des groupements hydroxyalkyle,

et répondant à la formule suivante:

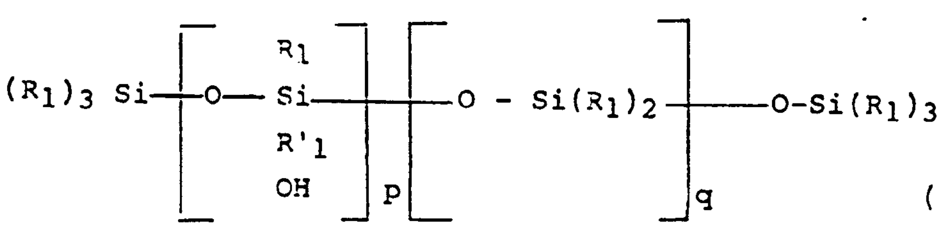

(I)

dans laquelle :

- les radicaux $R_1$, identiques ou différents, sont choisis parmi les radicaux méthyle et phényle au moins 60% en mole des radicaux $R_1$ étant méthyle;
- le radical $R'_1$ est un chaînon alkylène divalent hydrocarboné en $C_2$-$C_{18}$;
- p est compris entre 1 et 30 inclus;
- q est compris entre 1 et 150 inclus;

e/ des groupements alcoxylés;

f/ des groupements acyloxyalkyle répondant à la formule suivante:

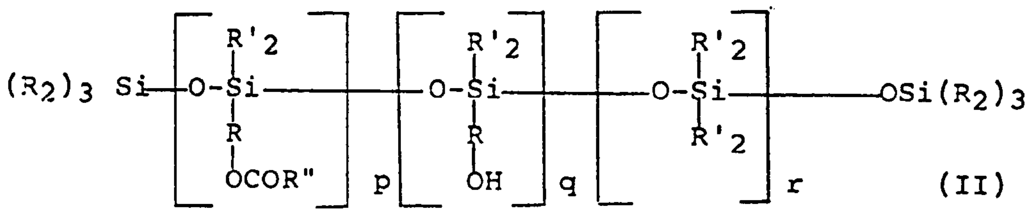

(II)

dans laquelle:

- $R_2$ désigne méthyle, phényle, $COOR_2$", hydroxyle, un seul des $R_2$ par atome de silicium peut être OH;
- $R'_2$ désigne méthyle, phényle, 60% molaire au moins de l'ensemble des radicaux $R_2$ et $R'_2$ est méthyle;
- R" désigne alcoyle ou alcénile en $C_8$-$C_{20}$;
- R designe un alkylène divalent hydrocarboné, linéaire ou ramifié, en $C_2$-$C_{18}$;
- r est compris entre 1 et 120 inclus;
- p est compris entre 1 et 30;
- q est 0 ou inférieur à 0,5 p, p+q étant compris entre 1 et 30;

les polymères de formule (II) pouvant contenir des groupements

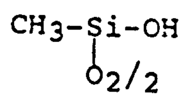

dans des proportions ne dépassant pas 15% de la somme p+q+r;

g/ des groupements diester, en particulier itaconate de diméthyle, répondant à la formule suivante:

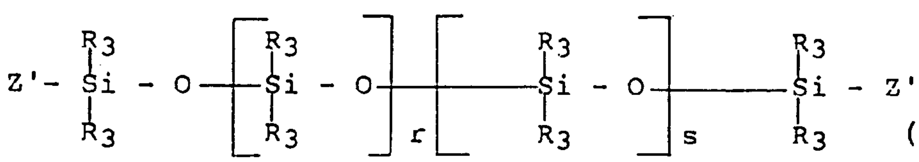

(III)

dans laquelle :

- les radicaux $R_3$, identiques ou différents, désignant un alkyle en $C_1$-$C_{20}$, vinyle, phényle, trifluoro-3,3,3 propyle ou hydroxyle, sous reserve qu'un seul radical $R_3$ peut désigner OH par atome de silicium;
- les symboles Z', identiques ou différents, désignent $R_3$ ou Z;
- Z est choisi parmi les groupes :

$CH_2$-CH ($COOR'_3$ )-$CH_2$-$COOR'_3$

$C(CH_3)(COOR'_3)$-$CH_2$-$COOR'_3$

- $R'_3$ désigne un radical hydrocarboné saturé, monovalent en $C_1$-$C_{12}$, un alcoxyalkyle, monovalent en $C_2$-$C_{12}$, un aryle, aralkyle ou alkylaryle en $C_6$-$C_{12}$;
- r est compris entre 0 et 500 inclus;
- s est compris entre 0 et 50 inclus;
- si s est égal à 0, un des symboles Z' est Z;

h/ des groupements ammonium quaternaire;

i/ des groupements amphotères ou bétaïniques;

j/ des groupements anioniques de type alkylcarboxylique 2-hydroxyalkylsulfonate ou 2-hydroxyalkylthiosulfate.

6. Utilisation selon l'une quelconque des revendications 1 à 5, caractérisée par le fait que l'organopolysiloxane est choisi parmi l'octaméthylcyclotétrasiloxane; le décaméthylcyclopentasiloxane; les polyalkylsiloxanes linéaires à groupements terminaux triméthylsilyle;les mélanges d'organosiloxanes et de silicones cycliques, la résine diméthyl/triméthylsiloxane; les polymères de formule (I), dans laquelle $R_1$ désigne méthyle et $R'_1$ un groupement triméthylène ou méthyl-2 triméthylène; les polymères de formule (II), dans laquelle $R_2$ et $R'_2$ désignent méthyle, R" un mélange de radicaux $C_{16}H_{33}$ et $C_{18}H_{37}$ et R désigne triméthylène ou méthyl-2 triméthylène; les polymères de formule (III), dans laquelle $R_3$ désigne $CH_3$ et Z' désigne OH.

7. Utilisation selon l'une quelconque des revendications 1 à 6, caractérisée par le fait que la dispersion aqueuse contient un copolymère d'acrylate d'ammonium/acrylamide (95/5 en poids), réticulé par un agent réticulant à polyinsaturation oléfinique pris dans le groupe constitué par le divinylbenzène, le tétraallyloxyéthane, l'éther diallylique, des éthers polyallylpolyglycéryliques et les éthers allyliques d'alcools de la série des sucres.

8. Utilisation selon l'une quelconque des revendications 1 à 7, caractérisée par le fait que l'organopolysiloxane est présent dans la dispersion dans des proportions comprises entre 0,5 et 50% en poids et de préférence entre 1 et 30% en poids et le copolymère acrylate d'ammonium/acrylamide est présent dans des proportions comprises entre 0,1 et 10% en poids et de préférence entre 0,1 et 4% en poids par rapport au poids total de la dispersion.

9. Utilisation selon l'une quelconque des revendications 1 à 8, caractérisée par le fait que la dispersion contient en plus au moins un agent émulsionnant non-ionique et une huile minérale.

10. Utilisation selon la revendication 9, caractérisée par le fait que l'agent émulsionnant non-ionique est choisi parmi les tensio-actifs oléosolubles dont le rapport hydrophile/lipophile est inférieur à 7.

11. Utilisation selon la revendication 9 ou 10, caractérisée par le fait que l'agent émulsionnant est constitué par un mélange de stéarate de sorbitan et d'un dérivé éthoxylé hydrophile.

12. Utilisation selon la revendication 9, caractérisée par le fait que l'huile minérale est l'huile de paraffine.

13. Utilisation selon l'une quelconque des revendications 9 à 12, caractérisée par le fait que l'agent émulsionnant non-ionique est présent dans des proportions comprises entre 0,01 et 10% en poids, et de préférence entre 0,01 et 0,6% en poids et l'huile minérale est présente dans des proportions comprises entre 0,08 et 6,5% en poids, et de préférence entre 0,08 et 2,5% en poids, les poids étant définis par rapport au poids total de la dispersion.

14. Utilisation selon l'une quelconque des revendications 1 à 13, caractérisée par le fait que le copolymère acrylate d'ammonium/acrylamide réticulé (95/5 en poids) est dispersé à raison de 30% en poids dans une émulsion eau-dans-huile constituée de 25% en poids de paraffine, 4% en poids de mélange de stéarate de sorbitan et de dérivé éthoxylé hydrophile et de 41% en poids d'eau.

15. Utilisation de la dispersion aqueuse selon l'une des revendications 1 à 14, caractérisée par le fait que l'émulsion eau-dans-huile contenant le copolymère acrylate d'ammonium/acrylamide réticulé (95/5 en poids) est présente dans la dispersion dans des proportions telles que la concentration en copolymère soit compris entre 0,05 et 10% en poids et de préférence entre 0.1 et 5% en poids de matière active en copolymère par rapport au poids total de la dispersion.

16. Composition cosmétique sous forme de dispersion aqueuse destinée au traitement des cheveux ou de la peau, caractérisée par le fait que la dispersion aqueuse est telle que définie dans l'une quelconque des revendications 1 à 15.

17. Composition selon la revendication 16, caractérisée par le fait qu'elle contient en plus des adjuvants habituellement utilisés en cosmétique, choisis parmi les parfums, les colorants, les conservateurs, les agents tensio-actifs anioniques, non-ioniques, amphotères ou cationiques, les séquestrants, les stabilisateurs de mousse, des polymères, des filtres solaires, des propulseurs, des substances actives sur le

plan cosmétique.

**18.** Composition selon l'une des revendications 16 ou 17, caractérisée par le fait qu'elle présente un pH compris entre 3 et 10 et de préférence entre 5 et 7.

**19.** Composition selon l'une quelconque des revendications 16 à 18, destinée au traitement des cheveux, caractérisée par le fait qu'elle se présente sous forme de shampooing, de produit à rincer, à appliquer avant ou après shampooing, avant ou après coloration ou décoloration, avant ou après permanente ou défrisage, ou comme produits de coiffage non rincés.

**20.** Composition selon l'une quelconque des revendications 16 à 18, destinée au traitement de la peau, caractérisée par le fait qu'elle se présente comme produit de bain ou de douche, de produit bronzant, de composition anti-solaire, de produit de rasage, de crème ou de lait pour le soin ou de lotion parfumée.

**21.** Procédé de traitement cosmétique des cheveux, caractérisé par le fait que l'on applique sur ceux-ci au moins une composition telle que définie dans la revendication 19.

**22.** Procédé de traitement cosmétique de la peau, caractérisé par le fait que l'on applique sur celle-ci une composition telle que définie dans la revendication 20.

**23.** Procédé de préparation d'une composition dermatologique sous forme de dispersion aqueuse, caractérisé par le fait que l'on incorpore dans une dispersion telle que définie dans l'une quelconque des revendications 1 à 15 dans une quantité efficace au moins une substance active sur le plan dermatologique.

**Revendications pour l'Etat contractant suivant : ES**

**1.** Utilisation en cosmétique, pour le traitement des cheveux ou de la peau d'une dispersion aqueuse, caractérisée par le fait que ladite dispersion contient au moins dans un milieu aqueux cosmétiquement ou physiologiquement acceptable, un organopolysiloxane et un copolymère d'acrylate d'ammonium/acrylamide réticulé.

**2.** Utilisation selon la revendication 1, caractérisée par le fait que l'organosiloxane est une silicone volatile ayant un point d'ébullition compris entre 60°C et 260°C, choisis parmi :

(i) les silicones cycliques de 3 à 7 atomes de silicium et de préférence 4 à 5 ou les cyclocopolymères du type diméthylsiloxane/méthylalkylsiloxane, de formule :

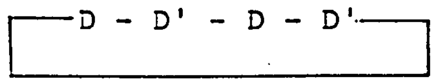

dans laquelle:

D représente

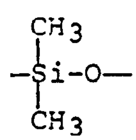

et D' représente :

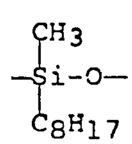

(ii) les silicones linéaires, ayant 2 à 9 atomes de silicium et possédant une viscosité inférieure ou égale à $5.10^{-6}$ $m^2/s$ à 25°C.

3. Utilisation selon l'une quelconque des revendications 1 à 2, caractérisée par le fait que l'organopolysiloxane est une silicone non volatile choisi parmi les polyalkylsiloxanes, les polyarylsiloxanes, les polyalkylarylsiloxanes, les copolymères polyéthersiloxanes, modifiés ou non, les gommes et résines de silicone, les polysiloxanes organomodifiés, ainsi que leurs mélanges.

4. Utilisation selon la revendication 3, caractérisé par le fait que l'organopolysiloxane est choisi parmi :
A/ les polyalkyl($C_1$-$C_{20}$)siloxanes, les polydiméhylsiloxanes linéaires à groupements terminaux triméthylsilyle, de viscosité $5.10^{-6}$ à $2{,}5.10^{-1}$ $m^2/s$ à 25°C et de préférence $10^{-5}$ à 1 $m^2/s$ , et les polydiméthylsiloxanes linéaires, à groupements terminaux trihydroxysilyle;
B/ les polydiméthylphénylsiloxanes, les polydiméthyldiphénylsiloxanes linéaires et/ou ramifiés, de viscosité $10^{-5}$ à $5.10^{-2}$ $m^2/s$ à 25°C;
C/ les copolymères d'oxyde d'éthylène et/ou de propylène avec un diorganosiloxane;
D/ les gommes de masse moléculaires comprise entre 200.000 et 1.000.000, utilisées seules ou sous forme de mélange dans un solvant, choisies dans le groupe constitué par les copolymères suivants :
- poly/(diméthyl siloxane)/(méthylvinylsiloxane)/
- poly/(diméthyl siloxane)/(diphénylsiloxane)/
- poly/(diméthyl siloxans)/(phénylméthyl siloxane)/
- poly/(diméthylsiloxant)/(diphénylsiloxant)/ (méthylvinylsiloxane)/;

et les mélanges suivants :
- les mélanges formés à partir d'un polydiméthylsiloxane hydroxylé en bout de chaîne et les polydiméthylsiloxanes cycliques;
- les mélanges formés à partir d'une gomme polydiméthylsiloxane et d'une silicone cyclique;
- les mélanges de deux polydiméthylsiloxanes de viscosités différentes;
- les mélange d'un polydiméthylsiloxane à chaîne latérale polyoxyéthylénée ou polyoxypropylénée et d'un polydiméthylsiloxane cyclique;

E/ les résines d'organopolysiloxanes constituées de systèmes siloxaniques réticulés, renfermant les unités $R_2SiO_{2/2}$,$RSiO_{3/2}$, $SiO_{4/2}$, dans lesquelles R représente un groupement hydrocarboné possédant 1 à 6 atomes de carbone ou un groupement phényle.

5. Utilisation selon la revendication 3, caractérisée par le fait que l'organopolysiloxane comporte dans sa structure générale un ou plusieurs groupement(s) organofonctionnel(s) directement fixé(s) sur la chaîne siloxanique ou fixé(s) par l'intermédiaire d'un radical hydrocarboné, et qu'il est choisi parmi les polyorganosiloxanes comportant :
a/ des groupements aminés substitués ou non;
b/ des groupements thiol;
c/ des groupements carboxylate;
d/ des groupements hydroxyalkyle,
et répondant à la formule suivante :

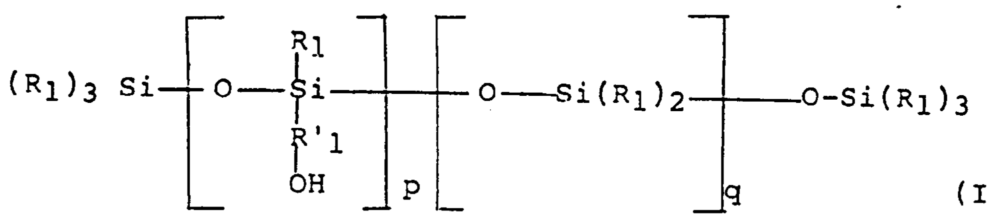

(I)

dans laquelle :
- les radicaux $R_1$, identiques ou différents, sont choisis parmi les radicaux méthyle et phényle, au moins 60% en mole des radicaux $R_1$ étant méthyle;
- le radical $R'_1$ est un chaînon alkylène divalent hydrocarboné en $C_2$-$C_{18}$;
- p est compris entre 1 et 30 inclus;
- q est compris entre 1 et 150 inclus;

e/ des groupements alcoxylés;
f/ des groupements acyloxyalkyle répondant à la formule suivante :

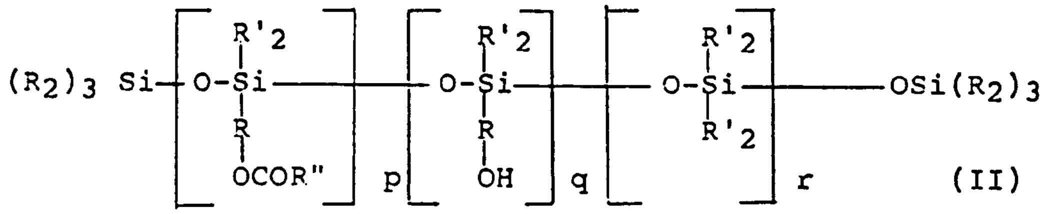

(II)

dans laquelle :

- $R_2$ désigne méthyle, phényle, $COOR_2$", hydroxyle, un seul des $R_2$ par atome de silicium peut être OH;
- $R'_2$ désigne méthyle, phényle, 60% molaire au moins de l'ensemble des radicaux $R_2$ et $R'_2$ est méthyle;
- R" désigne alcoyle ou alcényle en $C_8$-$C_{20}$;
- R désigne un alkylène divalent hydrocarboné, linéaire ou ramifié, en $C_2$-$C_{18}$;
- r est compris entre 1 et 120 inclus;
- p est compris entre 1 et 30;
- q est 0 ou inférieur à 0,5 p, p+q étant compris entre 1 et 30;

les polymères de formule (II) pouvant contenir des groupements

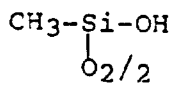

dans des proportions ne dépassant pas 15% de la somme p+q+r;

g/ des groupements diester, en particulier itaconate de diméthyle, répondant à la formule suivante :

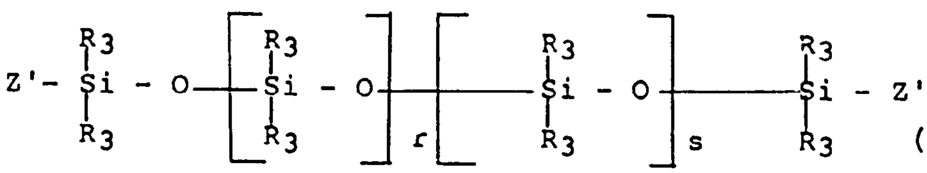

(III)

dans laquelle :

- les radicaux $R_3$, identiques ou différents, désignent un alkyle en $C_1$-$C_{20}$, vinyle, phényle, trifluoro-3,3,3 propyle ou hydroxyle, sous réserve qu'un seul radical $R_3$ peut désigner OH par atome de silicium;
- les symboles Z', identiques ou différents, désignent $R_3$ ou Z;
- Z est choisi parmi les groupes :

$$CH_2\text{-}CH\ (COOR'_3)\text{-}CH_2\text{-}COOR'_3$$
$$C(CH_3)(COOR'_3)\text{-}CH_2\text{-}COOR'_3$$

- $R'_3$ désigne un radical hydrocarboné saturé, monovalent en $C_1$-$C_{12}$, un alcoxyalkyle, monovalent en $C_2$-$C_{12}$ un aryle, aralkyle ou alkylaryle en $C_6$-$C_{12}$;
- r est compris entre 0 et 500 inclus;
- s est compris entre 0 et 50 inclus;
- si s est égal à 0, un des symboles Z' est Z;

h/ des groupements ammonium quaternaire;

i/ des groupements amphotères ou bétaïniques;

j/ des groupements anioniques de type alkylcarboxylique 2-hydroxyalkylsulfonate ou 2-hydroxyalkylthiosulfate.

**6.** Utilisation selon l'une quelconque des revendications 1 à 5, caractérisée par le fait que l'organopolysiloxane est choisi parmi l'octaméthylcyclotétrasiloxane; le décaméthylcyclopentasiloxane; les polyalkylsiloxanes linéaires à groupements terminaux triméthylsilyle; les mélanges d'organosiloxanes et de silicones cycliques, la résine diméthyl/triméthylsiloxane; les polymères de formule (I), dans laquelle $R_1$ désigne méthyle et $R'_1$ un groupement triméthylène ou méthyl-2 triméthylène; les polymères de formule (II), dans

laquelle $R_2$ et $R'_2$ désignent méthyle, R" un mélange de radicaux $C_{16}H_{33}$ et $C_{18}H_{37}$ et R désigne triméthylène ou méthyl-2 triméthylène; les polymères de formule (III), dans laquelle $R_3$ désigne $CH_3$ et Z' désigne OH.

**7.** Utilisation selon l'une quelconque des revendications 1 à 6, caractérisée par le fait que la dispersion aqueuse contient un copolymère d'acrylate d'ammonium/acrylamide (95/5 en poids), réticulé par un agent réticulant à polyinsaturation oléfinique pris dans le groupe constitué par le divinylbenzène, le tétraallyloxyéthane, l'éther diallylique, des éthers polyallylpolyglycèryliques et les éthers allyliques d'alcools de la série des sucres.

**8.** Utilisation selon l'une quelconque des revendications 1 à 7, caractérisée par le fait que l'organopolysiloxane est présent dans la dispersion dans des proportions comprises entre 0,5 et 50% en poids et de préférence entre 1 et 30% en poids et le copolymère acrylate d'ammonium/acrylamide est présent dans des proportions comprises entre 0,1 et 10% en poids et de préférence entre 0,1 et 4% en poids par rapport au poids total de la dispersion.

**9.** Utilisation selon l'une quelconque des revendications 1 à 8, caractérisée par le fait que la dispersion contient en plus au moins un agent émulsionnant non-ionique et une huile minérale.

**10.** Utilisation selon la revendication 9, caractérisée par le fait que l'agent émulsionnant non-ionique est choisi parmi les tensio-actifs oléosolubles dont le rapport hydrophile/lipophile est inférieur à 7.

**11.** Utilisation selon la revendication 9 ou 10, caractérisée par le fait que l'agent émulsionnant est constitué par un mélange de stéarate de sorbitan et d'un dérivé éthoxylé hydrophile.

**12.** Utilisation selon la revendication 9, caractérisée par le fait que l'huile minérale est l'huile de paraffine.

**13.** Utilisation selon l'une quelconque des revendications 9 à 12, caractérisée par le fait que l'agent émulsionnant non-ionique est présent dans des proportions comprises entre 0,01 et 10% en poids, et de préférence entre 0,01 et 0,6% en poids et l'huile minérale est présente dans des proportions comprises entre 0,08 et 6,5% en poids, et de préférence entre 0,08 et 2,5% en poids, les poids étant définis par rapport au poids total de la dispersion.

**14.** Utilisation selon l'une quelconque des revendications 1 à 13, caractérisée par le fait que le copolymère acrylate d'ammonium/acrylamide réticulé (95/5 en poids) est dispersé à raison de 30% en poids dans une émulsion eau-dans-huile constituée de 25% en poids de paraffine, 4% en poids de mélange de stéarate de sorbitan et de dérivé éthoxylé hydrophile et de 41% en poids d'eau.

**15.** Utilisation de la dispersion aqueuse selon l'une des revendications 1 à 14, caractérisée par le fait que l'émulsion eau-dans-huile contenant le copolymère acrylate d'ammonium/acrylamide réticulé (95/5 en poids) est présente dans la dispersion dans des proportions telles que la concentration en copolymère soit comprise entre 0,05 et 10% en poids et de préférence entre 0,1 et 5% en poids de matière active en copolymère par rapport au poids total de la dispersion.

**16.** Procédé de préparation d'une composition cosmétique destinée au traitement des cheveux ou de la peau caractérisé arp le fait que l'on utilise une dispersion aqueuse telle que définie dans l'une quelconque des revendications 1 à 15.

**17.** Composition cosmétique sous forme de dispersion aqueuse destinée au traitement des cheveux ou de la peau caractérisée par le fait que la dispersion aqueuse est telle que définie dans l'une quelconque des revendications 1 à 15.

**18.** Composition selon la revendication 17, caractérisée par le fait qu'elle contient en plus des adjuvants habituellement utilisées en cosmétique, choisis parmi les parfums, les colorants, les conservateurs, les agents tensio-actifs anionique, non-ioniques, amphotères ou cationiques, les séquestrants, les stabilisateurs de mousse, des polymères, des filtres solaires, des propulseurs, des substances actives sur le plan cosmétique.

**19.** Composition selon l'une des revendications 17 ou 18, caractérisée par le fait qu'elle présente un pH compris entre 3 et 10 et de préférence entre 5 et 7.

**20.** Composition selon l'une quelconque des revendications 17 à 19, destinée au traitement des cheveux, caractérisé par le fait qu'elle se présente sous forme de shampoing, de produit à rincer, à appliquer avant ou après shampooing, avant ou après coloration ou décoloration, avant ou après permanente ou défrisage, ou comme produits de coiffage non rincés.

**21.** Composition slon l'une quelconque des revendications 17 à 19, destinée au traitement de la peau, caractérisée par le fait qu'elle se présente comme produit de bain ou de douche, de produit bronzant, de composition anti-solaire, de produit de rasage, de crème ou de lait pour le soin ou de lotion parfumée.

**22.** Procédé de traitement cosmétique des cheveux, caractérisé par le fait que l'on applique sur ceux-ci au moins une composition telle que définie dans la revendication 20.

**23.** Procédé de traitement cosmétique de la peau, caractérisé par le fait que l'on applique sur celle-ci une composition telle que définie dans la revendication 21.

**24.** Procédé de préparation d'une composition dermatologique caractérisée par le fait que l'on utilise une dispersion aqueuse, telle que définie dans l'une quelconque des revendications 1 à 15 et qu'on incorpore dans celle-ci une quantité efficace d'au moins une substance active sur le plan dermatologique.

## Patentansprüche

### Patentansprüche für folgende Vertragsstaaten : AT, BE, CH, DE, DK, FR, GB, IT, LI, NL, SE

**1.** Kosmetische und/oder dermatologische Verwendung einer wässrigen Dispersion zur Behandlung der Haare oder der Haut, dadurch **gekennzeichnet**, daß
die genannte Dispersion mindestens in einem kosmetisch oder physiologisch zulässigen wässrigen Medium ein Organopolysiloxan und ein vernetztes Ammoniumacrylat/Acrylamid-Copolymer enthält.

**2.** Verwendung gemäß Anspruch 1,
dadurch **gekennzeichnet**, daß
das Organosiloxan ein flüchtiges Silicon mit einem Siedepunkt von 60 bis 260°C ist, ausgewählt aus:
(i) zyklischen Siliconen mit 3 bis 7 und vorzugsweise 4 bis 5 Siliziumatomen, oder Cyclocopolymeren des Typs Dimethylsiloxan/Methylalkylsiloxan der Formel:

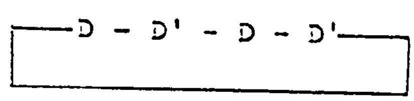

worin:
D darstellt:

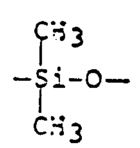

und D' darstellt:

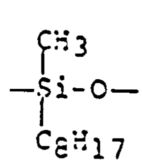

(ii) linearen Siliconen mit 2 bis 9 Siliziumatomen und einer Viskosität unterhalb oder gleich 5 x $10^{-6}$ $m^2/s$

bei 25°C.

3. Verwendung gemäß jedem Anspruch 1 und 2, dadurch **gekennzeichnet**, daß das Organopolysiloxan ein nichtflüchtiges Silicon ist, ausgewählt aus Polyalkylsiloxan, Polyarylsiloxanen, Polyalkylarylsiloxanen, modifizierten oder nicht modifizierten Polyethersiloxancopolymeren, Gummiprodukten und Harzen von Silicon, organomodifizierten Siloxanen, sowie aus deren Mischungen.

4. Vewendung gemäß Anspruch 3, dadurch **gekennzeichnet**, daß das Organopolysiloxan ausgewählt ist aus:
   A) Poly-$C_1$-$_{20}$-alkylsiloxanen, linearen Polydimethylsiloxanen mit endständigen Trimethylsilylgruppen, einer Viskosität von 5 x $10^{-6}$ bis 2,5 x $10^{-1}$ $m^2/s$ bei 25°C und vorzugsweise von $10^{-5}$ bis 1 $m^2/s$, und aus linearen Polydimethylsiloxanen mit endständigen Trihydroxysilylgruppen;
   B) Polydimethylphenylsiloxanen, linearen und/oder verzweigten Polydimethyldiphenylsiloxanen, einer Viskosität von $10^{-5}$ bis 5 x $10^{-2}$ $m^2/s$ bei 25°C;
   C) Copolymeren von Ethylen- und/oder Propylenoxid mit einem Diorganosiloxan;
   D) Gummiprodukten eines Molekulargewichts von 200 000 bis 1.000.000, alleine verwendet oder in Form einer Mischung in einem Lösungsmittel, ausgewählt aus der Gruppe aus den folgenden Copolymeren:
   - Poly[(dimethylsiloxan)/(methylvinylsiloxan)],
   - Poly[(dimethylsiloxan)/(diphenylsiloxan)],
   - Poly[(dimethylsiloxan)/(phenylmethylsiloxan)],
   - Poly[(dimethylsiloxan)/(diphenylsiloxan)/ (methylvinylsiloxan)],

   sowie aus folgenden Mischungen:
   - Mischungen aus einem am Kettenende hydroxylierten Polydimethylsiloxan und zyklischen Polydimethylsiloxanen;
   - Mischungen aus einem Polydimethylsiloxan-Gummi und einem zyklischen Silicon;
   - Mischungen aus zwei Polydimethylsiloxanen unterschiedlicher Viskositäten;
   - Mischungen aus einem Polydimethylsiloxan mit polyoxethylierter oder polyoxpropylierter Seitenkette und einem zyklischen Polydimethylsiloxan;

   E) Harzen von Organopolysiloxanen aus vernetzten Siloxansystemen, enthaltend die Einheiten $R_2SiO_2/_2$, $RSiO_{3/2}$, $SiO_4/_2$, worin R eine Kohlenwasserstoffgruppe mit 1 bis 6 Kohlenstoffatomen oder eine Phenylgrupe darstellt.

5. Verwendung gemäß Anspruch 3, dadurch **gekennzeichnet**, daß das Organopolysiloxan in seiner allgemeinen Struktur eine oder mehrere organofunktionelle Gruppen enthält, die direkt oder über das Zwischenglied eines Kohlenwasserstoffrestes an die Siloxankette gebunden ist (sind), und dass es aus Polyorganosiloxanen ausgewählt ist, enthaltend:
   a) substituierte oder nicht substituierte Amingruppen;
   b) Thiolgruppen;
   c) Carboxylatgruppen;
   d) Hydroxyalkylgruppen der folgenden Formel:

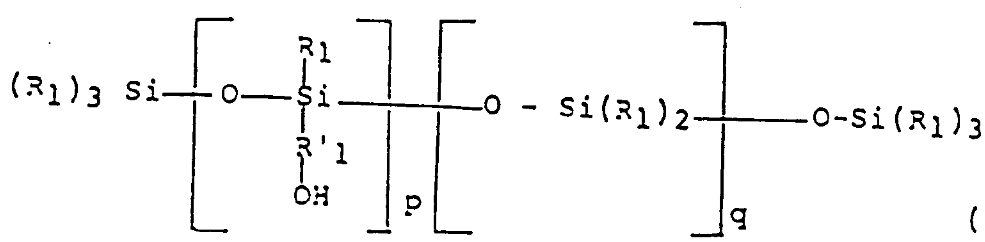

(I

   worin:
   - die Reste $R_1$,gleich oder verschieden, aus Methyl- und Phenylresten ausgewählt sind, wobei mindestens 60 Mol% der Reste $R_1$ Methylgruppen sind;

- der Rest R'$_1$ eine zweiwertige $C_{2-18}$-Alkylenkohlenwasserstoffkette ist;
- p 1 bis 30 beträgt;
- q 1 bis 150 beträgt;

e) Alkoxygruppen;

f) Acyloxyalkylgruppen der folgenden Folmel:

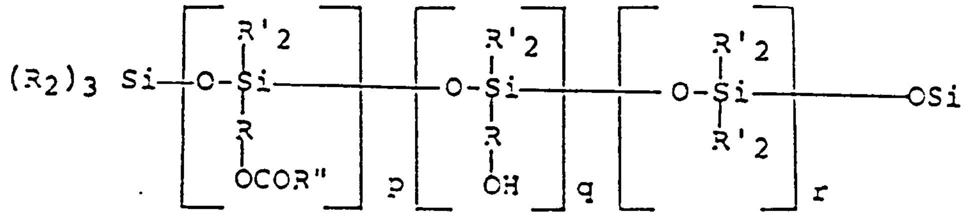

(II)

worin:
- $R_2$ einen Methyl-, Phenyl-, -COOR$_2$"-, Hydroxylrest bedeutet, wobei einer der Reste $R_2$ pro Siliziumatom OH sein kann;
- R'$_2$ einen Methyl- oder Phenylrest darstellt, wobei mindestens 60 Mol% aller Reste $R_2$ und R'$_2$ Methylreste sind;
- R" einen $C_8$-$_{20}$-Alkoyl- oder -Alkenylrest darstellt;
- R einen zweiwertigen linearen oder verzweigten $C_{2-18}$-Alkylenkohlenwasserstoffrest bedeutet;
- r 1 bis 120 beträgt;
- p 1 bis 30 beträgt;
- q 0 oder weniger als 0,5 p beträgt, wobei p+q 1 bis 30 beträgt; und wobei die Polyorganosiloxane der Formel (II) $CH_3$-Si-$O_2/_2$-OH-Gruppen in Mengenverhältnissen enthalten können, die 15% der Summe p+q+r nicht übersteigen;

g) Diestergruppen, insbesondere Dimethylitaconat, der folgenden Formel:

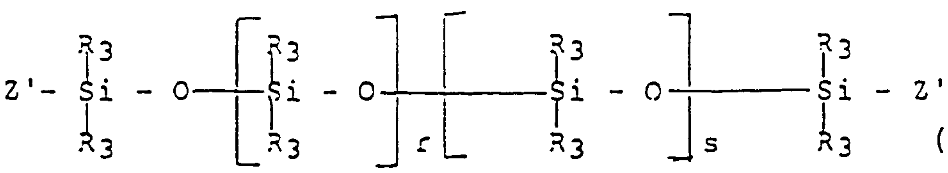

(III)

worin:
- die Reste $R_3$, gleich oder verschieden, $C_1$-$_{20}$-Alkyl, Vinyl, Phenyl, 3,3,3-Trifluorpropyl oder Hydroxyl bedeuten, mit der Maßgabe, daß einer der Reste $R_3$ eine OH-Gruppe pro Siliziumatom bedeuten kann;
- die Symbole Z', gleich oder verschieden, $R_3$ oder Z darstellen;
- Z aus den Gruppen ausgewählt ist:

$CH_2$-CH(COOR'$_3$)-$CH_2$-COOR'$_3$

C($CH_3$)(COOR'$_3$)-$CH_2$-COOR'$_3$
- R'$_3$ einen $C_1$-$_{12}$-Kohlenwasserstoffrest, einen einwertigen $C_2$-$_{12}$-Alkoxyalkylrest, $C_6$-$_{12}$-Aryl-, -Aralkyl- oder -Alkylarylrest darstellt;
- r 0 bis 500 beträgt,
- s 0 bis 50 beträgt;
- wenn s gleich 0 ist, eines der Symbole Z' Z ist;

h) quaternäre Ammoniumgruppen;

i) amphotere oder betainische Gruppen;

j) annionische Gruppen vom Typ Alkylcarboxyl-2-hydroxyalkylsulfonat oder -2-hydroxyalkylthiosulfat.

**6.** Verwendung gemäß jedem Anspruch 1 bis 5,
dadurch **gekennzeichnet**, daß
das Organopolysiloxan ausgewählt ist aus Octamethylcyclotetrasiloxan, Decamethylcyclopentasiloxan; linearen Polyalkylsiloxanen mit endständigen Trimethylsilylgruppen; Mischungen aus Organosiloxanen und zyklischen Siliconen, Dimethyl/Trimethylsiloxan-Harz; Polymeren der Formel (I), worin $R_1$ eine Me-

thylgruppe und $R'_1$ eine Trimethylen- oder 2-Methyltrimethylengruppe bedeuten; Polymeren der Formel (II), worin $R_2$ und $R'_2$ eine Methylgruppe, R" eine Mischung der Reste $C_{16}H_{33}$ und $C_{18}H_{37}$ und R eine Trimethylen- oder 2-Methyltrimethylengruppe bedeuten; Polymeren der Formel (III), worin $R_3$ $CH_3$ und Z' OH bedeuten.

**7.** Verbindung gemäß jedem Anspruch 1 bis 6,
dadurch **gekennzeichnet**, daß
die wässrige Dispersion ein Ammoniumacrylat/Acrylamid-Copolymer (95/5 an Gewicht) enthält, das durch ein Vernetzungsmittel mit olefinische Mehrfachungesättigtheit vernetzt ist, das ausgewählt ist aus der Gruppe aus Divinylbenzol, Tetrallyloxyethan, Diallylether, polyallylpolyglycerinethern und Allylethern von Alkoholen der Zuckerreihe.

**8.** Verwendung gemäß jedem Anspruch 1 bis 7,
dadurch **gekennzeichnet**, daß
das Organopolysiloxan in der Dispersion in Mengenverhältnissen von 0,5 bis 50, vorzugsweise 1 bis 30, Gew.%, und das Ammoniumacrylat/Acrylamid-Copolymer in Mengenverhältnissen von 0,1 bis 10, vorzugsweise 0,1 bis 4, Gew.%, bezogen auf Gesamtgewicht der Dispersion, vorliegen.

**9.** Verwendung gemäß jedem der Ansprüche 1 bis 8,
dadurch **gekennzeichnet**, daß
die Dispersion zusätzlich mindestens einen nicht-ionischen Emulgator und ein Mineralöl enthält.

**10.** Verwendung gemäß Anspruch 9,
dadurch **gekennzeichnet**, daß
der nicht-ionische Emulgator ausgewählt ist aus öllöslichen oberflächenaktiven Mitteln, deren hydrophiles/lipophiles Verhältnis einen Wert von unterhalb 7 hat.

**11.** Verwendung gemäß Anspruch 9 oder 10,
dadurch **gekennzeichnet**, daß
der Emulgator durch eine Mischung aus Sorbitanstearat und einem hydrophilen ethoxylierten Derivat dargestellt ist.

**12.** Verwendung gemäß Anspruch 9,
dadurch **gekennzeichnet**, daß
das Mineralöl Paraffinöl ist.

**13.** Verwendung gemäß jedem Anspruch 9 bis 12,
dadurch **gekennzeichnet**, daß
der nicht-ionische Emulgator in Mengenverhältnissen von 0,01 bis 10, vorzugsweise 0,01 bis 0,6, Gew.% und das Mineralöl in Mengenverhältnissen von 0,08 bis 6,5, vorzugsweise 0,08 bis 2,5, Gew.%, bezogen auf Gesamtgewicht der Dispersion, vorliegen.

**14.** Verwendung gemäß jedem Anspruch 1 bis 13,
dadurch **gekennzeichnet**, daß
das vernetzte Ammoniumacrylat /Acrylamid-Copolymer (95/5 an Gewicht) in einem Verhältnis von 30 Gew.% in einer Wasser-in-Öl-Emulsion aus 25 Gew.% Paraffin, 4 Gew.% Mischung aus Sorbitanstearat und hydrophilem ethoxylierten Derivat und 41 Gew.% Wasser dispergiert ist.

**15.** Verwendung der wässrigen Dispersion gemäß einem der Anspüche 1 bis 14,
dadurch **gekennzeichnet**, daß
die Wasser-in-Öl-Emulsion, enthaltend das vernetzte Ammoniumacrylat/Acrylamid-Copolymer (95/5 an Gewicht) in der Dispersion in solchen Mengenverhältnissen vorhanden ist, daß die Konzentration an Copolymer 0,05 bis 10, vorzugsweise 0,1 bis 5, Gew.% Aktivmasse an Copolymer, bezogen auf das Gesamtgewicht der Dispersion, beträgt.

**16.** Kosmetische Zusammensetzung in Form einer wässrigen Dispersion zur Behandlung der Haare oder der Haut,
dadurch **gekennzeichnet**, daß
die wässrige Dispersion wie in jedem der Ansprüche 1 bis 15 definiert ist.

17. Zusammensetzung gemäß Anspruch 16,
dadurch **gekennzeichnet**, daß
sie zusätzlich in der Kosmetik üblich verwendete Hilfstoffe enthält, ausgewählt aus Parfüms, Färbemitteln, Konservierungsmitteln, anionischen, nicht-ionischen, amphoteren oder kationischen oberflächenaktiven Mitteln, Sequestrierungsmitteln, Schaumstabilisatoren, Polymeren, Sonnenfiltermitteln, Treibmitteln, kosmetischen Wirksubstanzen.

18. Zusammensetzunge gemäß jedem Anspruch 16 oder 17,
dadurch **gekennzeichnet**, daß
sie einen pH von 3 bis 10 und vorzugsweise 5 bis 7 aufweist.

19. Zusammensetzung gemäß jedem Anspruch 16 bis 18 zur Behandlung der Haare,
dadurch **gekennzeichnet**, daß
sie in Form eines Shampoos, Produkts zur Spülung, zur Aufbringung vor oder nach dem Shampoonieren, vor oder nach der Färbung oder Entfärbung, vor oder nach der Dauerwelle oder dem Ausfrisieren, oder als nicht zur Spülung vorgesehene Frisurprodukte vorliegt.

20. Zusammensetzung gemäß jedem Anspruch 16 bis 18 zur Behandlung der Haut,
dadurch **gekennzeichnet**, daß
sie als Bade- oder Duschprodukt, Bräunungsmittel, Zusammensetzung zum Schutz vor schädlicher Sonneneinwirkung, Rasierprodukt, Creme oder Milch zur Pflege oder als parfümierte Lotion vorliegt.

21. Verfahren zur kosmetischen Behandlung der Haare,
dadurch **gekennzeichnet**, daß
man auf diese mindestens eine gemäß Anspruch 19 definierte Zusammensetzung aufträgt.

22. Verfahren zur ksometischen Behandlung der Haut,
dadurch **gekennzeichnet**, daß
man auf diese eine gemäß Anspruch 20 definierte Zusammensetzung aufträgt.

23. Dermatologische Zusammensetzung in Form einer wässrigen Dispersion,
dadurch **gekennzeichnet**, daß
die wässrige Dispersion wie in jedem der Ansprüche 1 bis 15 definiert ist und mindestens eine dermatologische Wirksubstanz in einer wirksamen Menge enthält.

**Patentansprüche für folgenden Vertragsstaat : GR**

1. Kosmetische und/oder dermatologische Verwendung einer wässrigen Dispersion zur Behandlung der Haare oder der Haut, dadurch **gekennzeichnet**, daß
die genannte Dispersion mindestens in einem kosmetisch oder physiologisch zulässigen wässrigen Medium ein Organopolysiloxan und ein vernetztes Ammoniumacrylat/Acrylamid-Copolymer enthält.

2. Verwendung gemäß Anspruch 1,
dadurch **gekennzeichnet**, daß
das Organosiloxan ein flüchtiges Silicon mit einem Siedepunkt von 60 bis 260°C ist, ausgewählt aus:
(i) zyklischen Siliconen mit 3 bis 7 und vorzugsweise 4 bis 5 Siliziumatomen, oder Cyclocopolymeren des Typs Dimethylsiloxan/Methylalkylsiloxan der Formel:

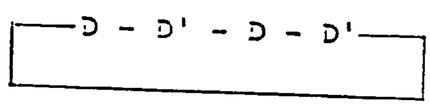

worin:
D darstellt:

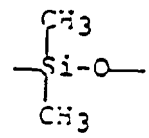

und D' darstellt:

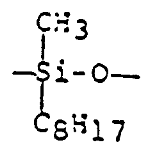

(ii) linearen Siliconen mit 2 bis 9 Siliziumatomen und einer Viskosität unterhalb oder gleich 5 x $10^{-6}$ $m^2/s$ bei 25°C.

**3.** Verwendung gemäß jedem Anspruch 1 und 2,
dadurch **gekennzeichnet**, daß
das Organopolysiloxan ein nichtflüchtiges Silicon ist, ausgewählt aus Polyalkylsiloxan, Polyarylsiloxanen, Polyalkylarylsiloxanen, modifizierten oder nicht modifizierten Polyethersiloxancopolymeren, Gummiprodukten und Harzen von Silicon, organomodifizierten Siloxanen, sowie aus deren Mischungen.

**4.** Vewendung gemäß Anspruch 3,
dadurch **gekennzeichnet**, daß
das Organopolysiloxan ausgewählt ist aus:
A) Poly-$C_1$-$_{20}$-alkylsiloxanen, linearen Polydimethylsiloxanen mit endständigen Trimethylsilylgruppen, einer Viskosität von 5 x $10^{-6}$ bis 2,5 x $10^{-1}$ $m^2/s$ bei 25°C und vorzugsweise von $10^{-5}$ bis 1 $m^2/s$, und aus linearen polydimethylsiloxanen mit endständigen Trihydroxysilylgruppen;
B) Polydimethylphenylsiloxanen, linearen und/oder verzweigten Polydimethyldiphenylsiloxanen, einer Viskosität von $10^{-5}$ bis 5 x $10^{-2}$ $m^2/s$ bei 25°C;
C) Copolymeren von Ethylen- und/oder Propylenoxid mit einem Diorganosiloxan;
D) Gummiprodukten eines Molekulargewichts von 200 000 bis 1.000.000, alleine verwendet oder in Form einer Mischung in einem Lösungsmittel, ausgewählt aus der Gruppe aus den folgenden Copolymeren:

- Poly[(dimethylsiloxan)/(methylvinylsiloxan)],
- Poly[(dimethylsiloxan)/(diphenylsiloxan)],
- Poly[(dimethylsiloxan)/(phenylmethylsiloxan)],
- Poly[(dimethylsiloxan)/(diphenylsiloxan)/ (methylvinylsiloxan)],

sowie aus folgenden Mischungen:

- Mischungen aus einem am Kettenende hydroxylierten Polydimethylsiloxan und zyklischen Polydimethylsiloxanen;
- Mischungen aus einem Polydimethylsiloxan-Gummi und einem zyklischen Silicon;
- Mischungen aus zwei Polydimethylsiloxanen unterschiedlicher Viskositäten;
- Mischungen aus einem Polydimethylsiloxan mit polyoxethylierter oder polyoxpropylierter Seitenkette und einem zyklischen Polydimethylsiloxan;

E) Harzen von Organopolysiloxanen aus vernetzten Siloxansystemen, enthaltend die Einheiten $R_2SiO_2/_2$, $RSiO_{3/2}$, $SiO_{4/2}$ worin R eine Kohlenwasserstoffgruppe mit 1 bis 6 Kohlenstoffatomen oder eine Phenylgrupe darstellt.

**5.** Verwendung gemäß Anspruch 3,
dadurch **gekennzeichnet**, daß
das Organopolysiloxan in seiner allgemeinen Struktur eine oder mehrere organofunktionelle Gruppen enthält, die direkt oder über das Zwischenglied eines Kohlenwasserstoffrestes an die Siloxankette gebunden ist (sind), und dass es aus Polyorganosiloxanen ausgewählt ist, enthaltend:
a) substituierte oder nicht substituierte Amingruppen;
b) Thiolgruppen;

c) Carboxylatgruppen;

d) Hydroxyalkylgruppen der folgenden Formel:

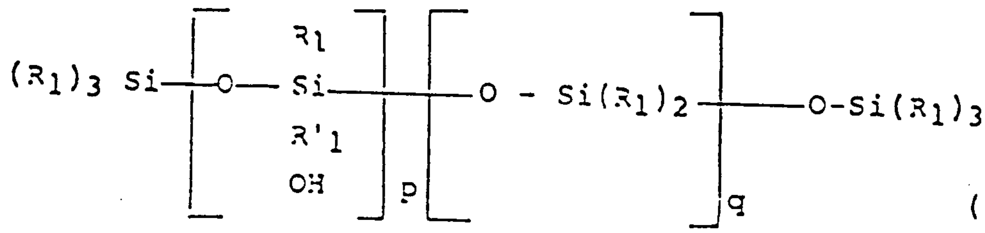

(I)

worin:

- die Reste $R_1$, gleich oder verschieden, aus Methyl- und Phenylresten ausgewählt sind, wobei mindestens 60 Mol% der Reste $R_1$ Methylgruppen sind;
- der Rest $R'_1$ eine zweiwertige $C_{2-18}$-Alkylenkohlenwasserstoffkette ist;
- p 1 bis 30 beträgt;
- q 1 bis 150 beträgt;

e) Alkoxygruppen;

f) Acyloxyalkylgrupppen der folgenden Formel:

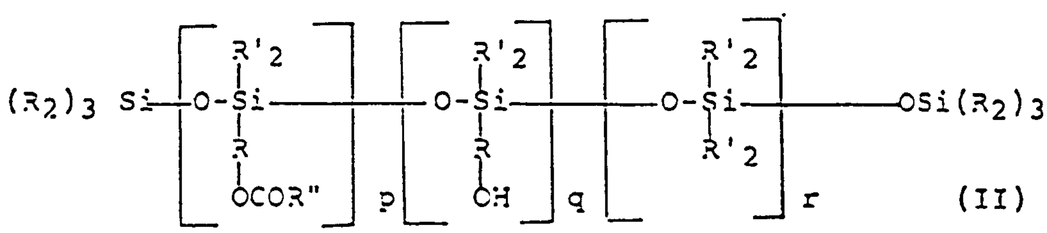

(II)

worin:

- $R_2$ einen Methyl-, Phenyl-, -$COOR_2$"-, Hydroxylrest bedeutet, wobei einer der Reste $R_2$ pro Siliziumatom OH sein kann;
- $R'_2$ einen Methyl- oder Phenylrest darstellt, wobei mindestens 60 Mol% aller Reste $R_2$ und $R'_2$ Methylreste sind;
- R" einen $C_{8-20}$-Alkoyl- oder -Alkenylrest darstellt;
- R einen zweiwertigen linearen oder verzweigten $C_{2-18}$-Alkylenkohlenwasserstoffrest bedeutet;
- r 1 bis 120 beträgt;
- p 1 bis 30 beträgt;
- q 0 oder weniger als 0,5 p beträgt, wobei p+q 1 bis 30 beträgt; und wobei die Polyorganosiloxane der Formel (II) $CH_3$-Si-$O_2/_2$-OH-Gruppen in Mengenverhältnissen enthalten können, die 15% der Summe p+q+r nicht übersteigen;

g) Diestergruppen, insbesondere Dimethylitaconat, der folgenden Formel:

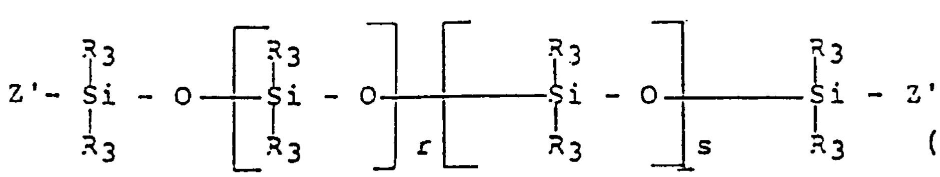

(III)

worin:

- die Reste $R_3$, gleich oder verschieden, $C_{1-20}$-Alkyl, Vinyl, Phenyl, 3,3,3-Trifluorpropyl oder Hydroxyl bedeuten, mit der Maßgabe, daß einer der Reste $R_3$ eine OH-Gruppe pro Siliziumatom bedeuten kann;
- die Symbole Z', gleich oder verschieden, $R_3$ oder Z darstellen;
- Z aus den Gruppen ausgewählt ist:

$$CH_2\text{-}CH(COOR'_3)\text{-}CH_2\text{-}COOR'_3$$

$C(CH_3)(COOR'_3)\text{-}CH_2\text{-}COOR'_3$

- $R'_3$ einen $C_{1-12}$-Kohlenwasserstoffrest, einen einwertigen $C_{2-12}$-Alkoxyalkylrest, $C_{6-12}$-Alkyl-, -Aralkyl- oder -Alkylarylrest darstellt;
- r 0 bis 500 beträgt,
- s 0 bis 50 beträgt;
- wenn s gleich 0 ist, eines der Symbole Z' Z ist;

h) quaternäre Ammoniumgruppen;
i) amphotere oder betainische Gruppen;
j) annionische Gruppen vom Typ Alkylcarboxyl-2-hydroxyalkylsulfonat oder -2-hydroxyalkylthiosulfat.

**6.** Verwendung gemäß jedem Anspruch 1 bis 5,
dadurch **gekennzeichnet**, daß
das Organopolysiloxan ausgewählt ist aus Octamethylcyclotetrasiloxan, Decamethylcyclopentasiloxan; linearen Polyalkylsiloxanen mit endständigen Trimethylsilylgruppen; Mischungen aus Organosiloxanen und zyklischen Siliconen, Dimethyl/Trimethylsiloxan-Harz; Polymeren der Formel (I), worin $R_1$ eine Methylgruppe und $R'_1$ eine Trimethylen- oder 2-Methyltrimethylengruppe bedeuten; Polymeren der Formel (II), worin $R_2$ und $R'_2$ eine Methylgruppe, R" eine Mischung der Reste $C_{16}H_{33}$ und $C_{18}H_{37}$ und R eine Trimethylen- oder 2-Methyltrimethylengruppe bedeuten; Polymeren der Formel (III), worin $R_3$ $CH_3$ und Z' OH bedeuten.

**7.** Verbindung gemäß jedem Anspruch 1 bis 6,
dadurch **gekennzeichnet**, daß
die wässrige Dispersion ein Ammoniumacrylat/Acrylamid-Copolymer (95/5 an Gewicht) enthält, das durch ein Vernetzungsmittel mit olefinischer Mehfachungesättigtheit vernetzt ist, das ausgewählt ist aus der Gruppe aus Divinylbenzol, Tetrallyloxyethan, Diallylether, Polyallylpolyglycerinethern und Allylethern von Alkoholen der Zuckerreihe.

**8.** Verwendung gemäß jedem Anspruch 1 bis 7,
dadurch **gekennzeichnet**, daß
das Organopolysiloxan in der Dispersion in Mengenverhältnissen von 0,5 bis 50, vorzugsweise 1 bis 30, Gew.% und das Ammoniumacrylat/Acrylamid-Copolymer in Mengenverhältnissen von 0,1 bis 10, vorzugsweise 0,1 bis 4, Gew.%, bezogen auf Gesamtgewicht der Dispersion, vorliegen.

**9.** Verwendung gemäß jedem der Ansprüche 1 bis 8,
dadurch **gekennzeichnet**, daß
die Dispersion zusätzlich mindestens einen nicht-ionischen Emulgator und ein Mineralöl enthält.

**10.** Verwendung gemäß Anspruch 9,
dadurch **gekennzeichnet**, daß
der nicht-ionische Emulgator ausgewählt ist aus öllöslichen oberflächenaktiven Mitteln, deren hydrophiles/lipophiles Verhältnis einen Wert von unterhalb 7 hat.

**11.** Verwendung gemäß Anspruch 9 oder 10,
dadurch **gekennzeichnet**, daß
der Emulgator durch eine Mischung aus Sorbitanstearat und einem hydrophilen ethoxylierten Derivat dargestellt ist.

**12.** Verwendung gemäß Anspruch 9,
dadurch **gekennzeichnet**, daß
das Mineralöl Paraffinöl ist.

**13.** Verwendung gemäß jedem Anspruch 9 bis 12,
dadurch **gekennzeichnet**, daß
der nicht-ionische Emulgator in Mengenverhältnissen von 0,01 bis 10, vorzugsweise 0,01 bis 0,6, Gew.% und das Mineralöl in Mengenverhältnissen von 0,08 bis 6,5, vorzugsweise 0,08 bis 2,5, Gew.%, bezogen auf Gesamtgewicht der Dispersion, vorliegen.

**14.** Verwendung gemäß jedem Anspruch 1 bis 13,
dadurch **gekennzeichnet**, daß

das vernetzte Ammoniumacrylat /Acrylamid-Copolymer (95/5 an Gewicht) in einem Verhältnis von 30 Gew.% in einer Wasser-in-Öl-Emulsion aus 25 Gew.% Paraffin, 4 Gew.% Mischung aus Sorbitanstearat und hydrophilem ethoxylierten Derivat und 41 Gew.% Wasser dispergiert ist.

**15.** Verwendung der wässrigen Dispersion gemäß einem der Anspüche 1 bis 14, dadurch **gekennzeichnet**, daß die Wasser-in-Öl-Emulsion, enthaltend das vernetzte Ammoniumacrylat/Acrylamid-Copolymer (95/5 an Gewicht) in der Dispersion in solchen Mengenverhältnissen vorhanden ist, daß die Konzentration an Copolymer 0,05 bis 10, vorzugsweise 0,1 bis 5, Gew.% Aktivmasse an Copolymer, bezogen auf das Gesamtgewicht der Dispersion, beträgt.

**16.** Kosmetische Zusammensetzung in Form einer wässrigen Dispersion zur Behandlung der Haare oder der Haut, dadurch **gekennzeichnet**, daß die wässrige Dispersion wie in jedem der Ansprüche 1 bis 15 definiert ist.

**17.** Zusammensetzung gemäß Anspruch 16, dadurch **gekennzeichnet**, daß sie zusätzlich in der Kosmetik üblich verwendete Hilfstoffe enthält, ausgewählt aus Parfüms, Färbemitteln, Konservierungsmitteln, anionischen, nicht-ionischen, amphoteren oder kationischen oberflächenaktiven Mitteln, Sequestrierungsmitteln, Schaumstabilisatoren, Polymeren, Sonnenfiltermitteln, Treibmitteln, kosmetischen Wirksubstanzen.

**18.** Zusammensetzunge gemäß jedem Anspruch 16 oder 17, dadurch **gekennzeichnet**, daß sie einen pH von 3 bis 10 und vorzugsweise 5 bis 7 aufweist.

**19.** Zusammensetzung gemäß jedem Anspruch 16 bis 18 zur Behandlung der Haare, dadurch **gekennzeichnet**, daß sie in Form eines Shampoos, Produkts zur Spülung, zur Aufbringung vor oder nach dem Shampoonieren, vor oder nach der Färbung oder Entfärbung, vor oder nach der Dauerwelle oder dem Ausfrisieren, oder als nicht zur Spülung vorgesehene Frisurprodukte vorliegt.

**20.** Zusammensetzung gemäß jedem Anspruch 16 bis 18 zur Behandlung der Haut, dadurch **gekennzeichnet**, daß sie als Bade- oder Duschprodukt, Bräunungsmittel, Zusammensetzung zum Schutz vor schädlicher Sonneneinwirkung, Rasierprodukt, Creme oder Milch zur Pflege oder als parfümierte Lotion vorliegt.

**21.** Verfahren zur kosmetischen Behandlung der Haare, dadurch **gekennzeichnet**, daß man auf diese mindestens eine gemäß Anspruch 19 definierte Zusammensetzung aufträgt.

**22.** Verfahren zur ksometischen Behandlung der Haut, dadurch **gekennzeichnet**, daß man auf diese eine gemäß Anspruch 20 definierte Zusammensetzung aufträgt.

**23.** Verfahren zur Herstellung einer dermatologischen Zusammensetzung in Form einer wässrigen Dispersion, dadurch **gekennzeichnet**, daß man einer wie in jedem der Ansprüche 1 bis 15 definierten Dispersion eine wirksame Menge mindestens einer dermatologischen Wirksubstanz einverleibt.

**Patentansprüche für folgenden Vertragsstaat : ES**

**1.** Kosmetische und/oder dermatologische Verwendung einer wässrigen Dispersion zur Behandlung der Haare oder der Haut, dadurch **gekennzeichnet**, daß die genannte Dispersion mindestens in einem kosmetisch oder physiologisch zulässigen wässrigen Medium ein Organopolysiloxan und ein vernetztes Ammoniumacrylat/Acrylamid-Copolymer enthält.

2. Verwendung gemäß Anspruch 1, dadurch **gekennzeichnet**, daß das Organosiloxan ein flüchtiges Silicon mit einem Siedepunkt von 60 bis 260°C ist, ausgewählt aus:

(i) zyklischen Siliconen mit 3 bis 7 und vorzugsweise 4 bis 5 Siliziumatomen, oder Cyclocopolymeren des Typs Dimethylsiloxan/Methylalkylsiloxan der Formel:

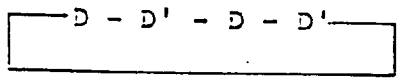

worin:

D darstellt:

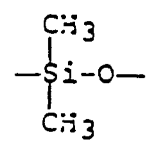

und D' darstellt:

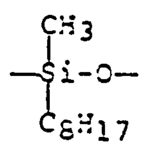

(ii) linearen Siliconen mit 2 bis 9 Siliziumatomen und einer Viskosität unterhalb oder gleich 5 x $10^{-6}$ $m^2/s$ bei 25°C.

3. Verwendung gemäß jedem Anspruch 1 und 2, dadurch **gekennzeichnet**, daß das Organopolysiloxan ein nichtflüchtiges Silicon ist, ausgewählt aus Polyalkylsiloxan, Polyarylsiloxanen, Polyalkylarylsiloxanen, modifizierten oder nicht modifizierten Polyethersiloxancopolymeren, Gummiprodukten und Harzen von Silicon, organomodifizierten Siloxanen, sowie aus deren Mischungen.

4. Vewendung gemäß Anspruch 3, dadurch **gekennzeichnet**, daß das Organopolysiloxan ausgewählt ist aus:

A) Poly-$C_{1-20}$-alkylsiloxanen, linearen polydimethylsiloxanen mit endständigen Trimethylsilylgruppen, einer Viskosität von 5 x $10^{-6}$ bis 2,5 x $10^{-1}$ $m^2/s$ bei 25°C und vorzugsweise von $10^{-5}$ bis 1 $m^2/s$, und aus linearen Polydimethylsiloxanen mit endständigen Trihydroxysilylgruppen;

B) Polydimethylphenylsiloxanen, linearen und/oder verzweigten Polydimethyldiphenylsiloxanen, einer Viskosität von $10^{-5}$ bis 5 x $10^{-2}$ $m^2/s$ bei 25°C;

C) Copolymeren von Ethylen- und/oder Propylenoxid mit einem Diorganosiloxan;

D) Gummiprodukten eines Molekulargewichts von 200 000 bis 1.000.000, alleine verwendet oder in Form einer Mischung in einem Lösungsmittel, ausgewählt aus der Gruppe aus den folgenden Copolymeren:

- Poly[(dimethylsiloxan)/(methylvinylsiloxan)],
- Poly[(dimethylsiloxan)/(diphenylsiloxan)],
- Poly[(dimethylsiloxan)/(phenylmethylsiloxan)],
- Poly[(dimethylsiloxan)/(diphenylsiloxan)/ (methylvinylsiloxan)],

sowie aus folgenden Mischungen:

- Mischungen aus einem am Kettenende hydroxylierten Polydimethylsiloxan und zyklischen Polydimethylsiloxanen;
- Mischungen aus einem Polydimethylsiloxan-Gummi und einem zyklischen Silicon;
- Mischungen aus zwei Polydimethylsiloxanen unterschiedlicher Viskositäten;

- Mischungen aus einem Polydimethylsiloxan mit polyoxethylierter oder polyoxpropylierter Seitenkette und einem zyklischen Polydimethylsiloxan;

E) Harzen von Organopolysiloxanen aus vernetzten Siloxansystemen, enthaltend die Einheiten $R_2SiO_{2/2}$, $RSiO_{3/2}$, $SiO_{4/2}$, worin R eine Kohlenwasserstoffgruppe mit 1 bis 6 Kohlenstoffatomen oder eine Phenylgrupe darstellt.

**5.** Verwendung gemäß Anspruch 3,
dadurch **gekennzeichnet**, daß
das Organopolysiloxan in seiner allgemeinen Struktur eine oder mehrere organofunktionelle Gruppen enthält, die direkt oder über das Zwischenglied eines Kohlenwasserstoffrestes an die Siloxankette gebunden ist (sind), und dass es aus Polyorganosiloxanen ausgewählt ist, enthaltend:

a) substituierte oder nicht substituierte Amingruppen;
b) Thiolgruppen;
c) Carboxylatgruppen;
d) Hydroxyalkylgruppen der folgenden Formel:

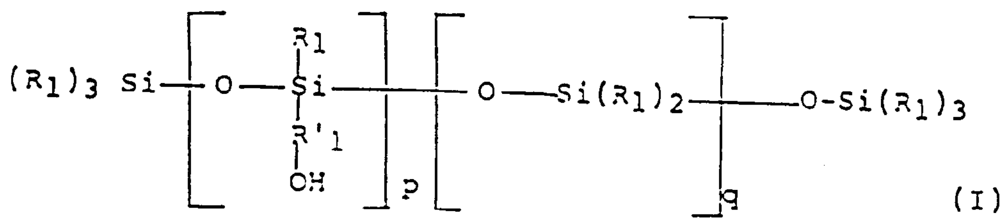

(I)

worin:

- die Reste $R_1$, gleich oder verschieden, aus Methyl- und Phenylresten ausgewählt sind, wobei mindestens 60 Mol% der Reste $R_1$ Methylgruppen sind;
- der Rest $R'_1$ eine zweiwertige $C_{2-18}$-Alkylenkohlenwasserstoffkette ist;
- p 1 bis 30 beträgt;
- q 1 bis 150 beträgt;

e) Alkoxygruppen;
f) Acyloxyalkylgrupppen der folgenden Formel:

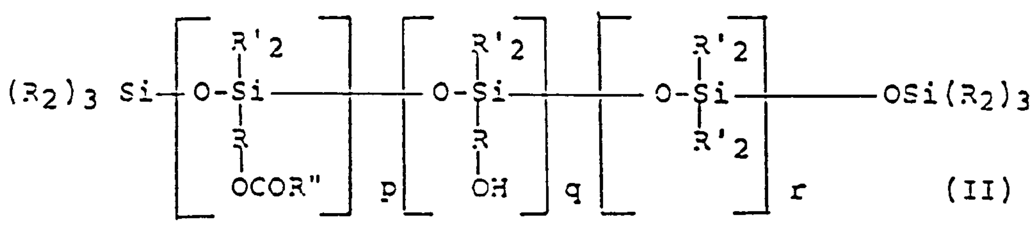

(II)

worin:

- $R_2$ einen Methyl-, Phenyl-, -$COOR_2$"-, Hydroxylrest bedeutet, wobei einer der Reste $R_2$ pro Siliziumatom OH sein kann;
- $R'_2$ einen Methyl- oder Phenylrest darstellt, wobei mindestens 60 Mol% aller Reste $R_2$ und $R'_2$ Methylreste sind;
- R" einen $C_{8-20}$-Alkoyl- oder -Alkenylrest darstellt;
- R einen zweiwertigen linearen oder verzweigten $C_{2-18}$-Alkylenkohlenwasserstoffrest bedeutet;
- r 1 bis 120 beträgt;
- p 1 bis 30 beträgt;
- q 0 oder weniger als 0,5 p beträgt, wobei p+q 1 bis 30 beträgt; und wobei die Polyorganosiloxane der Formel (II) $CH_3$-Si-$O_{2/2}$-OH-Gruppen in Mengenverhältnissen enthalten können, die 15% der Summe p+q+r nicht übersteigen;

g) Diestergruppen, insbesondere Dimethylitaconat, der folgenden Formel:

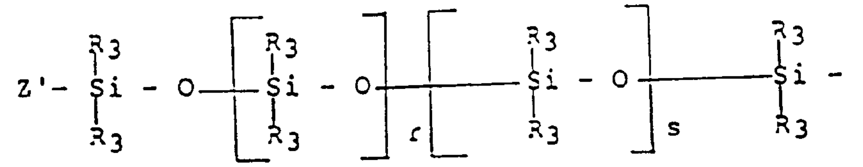

(III)

worin:

- die Reste $R_3$, gleich oder verschieden, $C_{1-20}$-Alkyl, Vinyl, Phenyl, 3,3,3-Trifluorpropyl oder Hydroxyl bedeuten, mit der Maßgabe, daß einer der Reste $R_3$ eine OH-Gruppe pro Siliziumatom bedeuten kann;
- die Symbole Z', gleich oder verschieden, $R_3$ oder Z darstellen;
- Z aus den Gruppen ausgewählt ist:

  $CH_2$-CH(COOR'$_3$)-$CH_2$-COOR'$_3$

  C($CH_3$)(COOR'$_3$)-$CH_2$-COOR'$_3$
- R'$_3$ einen $C_{1-12}$-Kohlenwasserstoffrest, einen einwertigen $C_{2-12}$-Alkoxyalkylrest, $C_{6-12}$-Aryl-, -Aralkyl- oder -Alkylarylrest darstellt;
- r 0 bis 500 beträgt,
- s 0 bis 50 beträgt;
- wenn s gleich 0 ist, eines der Symbole Z' Z ist;

h) quaternäre Ammoniumgruppen;
i) amphotere oder betainische Gruppen;
j) annionische Gruppen vom Typ Alkylcarboxyl-2-hydroxyalkylsulfonat oder -2-hydroxyalkylthiosulfat.

**6.** Verwendung gemäß jedem Anspruch 1 bis 5,
dadurch **gekennzeichnet**, daß
das Organopolysiloxan ausgewählt ist aus Octamethylcyclotetrasiloxan, Decamethylcyclopentasiloxan; linearen Polyalkylsiloxanen mit endständigen Trimethylsilylgruppen; Mischungen aus Organosiloxanen und zyklischen Siliconen, Dimethyl/Trimethylsiloxan-Harz; Polymeren der Formel (I), worin $R_1$ eine Methylgruppe und $R'_1$ eine Trimethylen- oder 2-Methyltrimethylengruppe bedeuten; Polymeren der Formel (II), worin $R_2$ und $R'_2$ eine Methylgruppe, R" eine Mischung der Reste $C_{16}H_{33}$ und $C_{18}H_{37}$ und R eine Trimethylen- oder 2-Methyltrimethylengruppe bedeuten; Polymeren der Formel (III), worin $R_3$ $CH_3$ und Z' OH bedeuten.

**7.** Verbindung gemäß jedem Anspruch 1 bis 6,
dadurch **gekennzeichnet**, daß
die wässrige Dispersion ein Ammoniumacrylat/Acrylamid-Copolymer (95/5 an Gewicht) enthält, das durch ein Vernetzungsmittel mit olefinischer Mehrfachungesättigtheit vernetzt ist, das ausgewählt ist aus der Gruppe aus Divinylbenzol, Tetrallyloxyethan, Diallylether, Polyallylpolyglycerinethern und Allylethern von Alkoholen der Zuckerreihe.

**8.** Verwendung gemäß jedem Anspruch 1 bis 7,
dadurch **gekennzeichnet**, daß
das Organopolysiloxan in der Dispersion in Mengenverhältnissen von 0,5 bis 50, vorzugsweise 1 bis 30, Gew.% und das Ammoniumacrylat/Acrylamid-Copolymer in Mengenverhältnissen von 0,1 bis 10, vorzugsweise 0,1 bis 4, Gew.%, bezogen auf Gesamtgewicht der Dispersion, vorliegen.

**9.** Verwendung gemäß jedem der Ansprüche 1 bis 8,
dadurch **gekennzeichnet**, daß
die Dispersion zusätzlich mindestens einen nicht-ionischen Emulgator und ein Mineralöl enthält.

**10.** Verwendung gemäß Anspruch 9,
dadurch **gekennzeichnet**, daß
der nicht-ionische Emulgator ausgewählt ist aus öllöslichen oberflächenaktiven Mitteln, deren hydrophiles/lipophiles Verhältnis einen Wert von unterhalb 7 hat.

**11.** Verwendung gemäß Anspruch 9 oder 10,

dadurch **gekennzeichnet**, daß
der Emulgator durch eine Mischung aus Sorbitanstearat und einem hydrophilen ethoxylierten Derivat dargestellt ist.

**12.** Verwendung gemäß Anspruch 9,
dadurch **gekennzeichnet**, daß
das Mineralöl paraffinöl ist.

**13.** Verwendung gemäß jedem Anspruch 9 bis 12,
dadurch **gekennzeichnet**, daß
der nicht-ionische Emulgator in Mengenverhältnissen von 0,01 bis 10, vorzugsweise 0,01 bis 0,6, Gew.% und das Mineralöl in Mengenverhältnissen von 0,08 bis 6,5, vorzugsweise 0,08 bis 2,5, Gew.%, bezogen auf Gesamtgewicht der Dispersion, vorliegen.

**14.** Verwendung gemäß jedem Anspruch 1 bis 13,
dadurch **gekennzeichnet**, daß
das vernetzte Ammoniumacrylat /Acrylamid-Copolymer (95/5 an Gewicht) in einem Verhältnis von 30 Gew.% in einer Wasser-in-Öl-Emulsion aus 25 Gew.% Paraffin, 4 Gew.% Mischung aus Sorbitanstearat und hydrophilem ethoxylierten Derivat und 41 Gew.% Wasser dispergiert ist.

**15.** Verwendung der wässrigen Dispersion gemäß einem der Anspüche 1 bis 14,
dadurch **gekennzeichnet**, daß
die Wasser-in-Öl-Emulsion, enthaltend das vernetzte Ammoniumacrylat/Acrylamid-Copolymer (95/5 an Gewicht) in der Dispersion in solchen Mengenverhältnissen vorhanden ist, daß die Konzentration an Copolymer 0,05 bis 10, vorzugsweise 0,1 bis 5, Gew.% Aktivmasse an Copolymer, bezogen auf das Gesamtgewicht der Dispersion, beträgt.

**16.** Verfahren zur Herstellung einer kosmetischen Zusammensetzung zur Behandlung der Haare oder der Haut,
dadurch **gekennzeichnet**, daß
man eine wie in jedem der Ansprüche 1 bis 15 definierte wässrige Dispersion verwendet.

**17.** Kosmetische Zusammensetzung in Form einer wässrigen Dispersion zur Behandlung der Haare oder der Haut,
dadurch **gekennzeichnet**, daß
die wässrige Dispersion wie in jedem der Ansprüche 1 bis 15 definiert ist.

**18.** Zusammensetzung gemäß Anspruch 17,
dadurch **gekennzeichnet**, daß
sie zusätzlich in der Kosmetik üblich verwendete Hilfstoffe enthält, ausgewählt aus Parfüme, Färbemitteln, Konservierungsmitteln, anionischen, nicht-ionischen, amphoteren oder kationischen oberflächenaktiven Mitteln, Sequestrierungsmitteln, Schaumstabilisatoren, Polymeren, Sonnenfiltermitteln, Treibmitteln, kosmetischen Wirksubstanzen.

**19.** Zusammensetzung gemäß jedem Anspruch 17 oder 18,
dadurch **gekennzeichnet**, daß
sie einen pH von 3 bis 10 und vorzugsweise 5 bis 7 aufweist.

**20.** Zusammensetzung gemäß jedem Anspruch 17 bis 19 zur Behandlung der Haare,
dadurch **gekennzeichnet**, daß
sie in Form eines Shampoos, Produkts zur Spülung, zur Aufbringung vor oder nach dem Shampoonieren, vor oder nach der Färbung oder Entfärbung, vor oder nach der Dauerwelle oder dem Ausfrisieren, oder als nicht zur Spülung vorgesehene Frisurprodukte vorliegt.

**21.** Zusammensetzung gemäß jedem Anspruch 17 bis 19 zur Behandlung der Haut,
dadurch **gekennzeichnet**, daß
sie als Bade- oder Duschprodukt, Bräunungsmittel, Zusammenseztung zum Schutz vor schädlicher Sonneneinwirkung, Rasierprodukt, Creme oder Milch zur Pflege oder als parfümierte Lotion vorliegt.

22. Verfahren zur kosmetischen Behandlung der Haare, dadurch **gekennzeichnet**, daß man auf diese mindestens eine gemäß Anspruch 20 definierte Zusammensetzung aufträgt.

23. Verfahren zur kosmetischen Behandlung der Haut, dadurch **gekennzeichnet**, daß man auf diese eine gemäß Anspruch 21 definierte Zusammensetzung aufträgt.

24. Verfahren zur Herstellung einer dermatologischen Zusammensetzung, dadurch **gekennzeichnet**, daß man eine wie in jedem der Ansprüche 1 bis 15 definierte wässrige Dispersion verwendet und dieser eine wirksame Menge mindestens einer dermatologischen Wirksubstanz einverleibt.

## Claims

**Claims for the following Contracting States : AT, BE, CH, DE, DK, FR, GB, IT, LI, NL, SE**

1. Use of an aqueous dispersion in cosmetics, for the treatment of hair or the skin and/or in dermatology, characterized in that the said dispersion contains an organopolysiloxane and a crosslinked ammonium acrylate/acrylamide copolymer at least in a cosmetically or physiologically acceptable aqueous medium.

2. Use according to Claim 1, characterized in that the organosiloxane is a volatile silicone which has a boiling point of between 60°C and 260°C, chosen from:
(i) cyclic silicones containing 3 to 7 and preferably 4 to 5 silicon atoms or cyclocopolymers of the dimethylsiloxane/methylalkylsiloxane type, of formula:

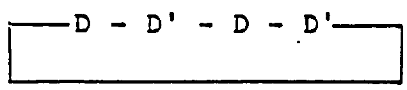

in which:
D denotes:

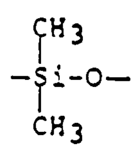

and D' denotes:

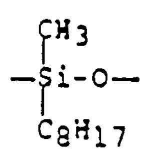

(ii) linear silicones containing 2 to 9 silicon atoms and having a viscosity lower than or equal to $5x10^{-6}$ $m^2/s$ at 25°C.

3. Use according to either of Claims 1 to 2, characterized in that the organopolysiloxane is a nonvolatile silicone chosen from polyalkylsiloxanes, polyarylsiloxanes, polyalkylarylsiloxanes, polyethersiloxane copolymers, modified or otherwise, silicone gums and resins, organomodified polysiloxanes and mixtures thereof.

4. Use according to Claim 3, characterized in that the organopolysiloxane is chosen from:

A) polyalkyl($C_1$-$C_{20}$)siloxanes, linear polydimethylsiloxanes containing trimethylsilyl end groups, with a viscosity of $5x10^{-6}$ to $2.5x10^{-1}$ $m^2/s$ at 25°C and preferably $10^{-5}$ to 1 $m^2/s$ and linear polydimethylsiloxanes containing trihydroxysilyl end groups;

B) linear and/or branched polydimethylphenylsiloxanes and polydimethyldiphenylsiloxanes with a viscosity of $10^{-5}$ to $5x10^{-2}$ $m^2/s$ at 25°C;

C) copolymers of propylene and/or ethylene oxide with a diorganosiloxane;

D) gums with a molecular mass between 200,000 and 1,000,000, employed by themselves or in the form of a mixture in a solvent, which are chosen from the group consisting of the following copolymers:

- poly[(dimethylsiloxane)/(methylvinylsiloxane)]
- poly[(dimethylsiloxane)/(diphenylsiloxane)]
- poly[(dimethylsiloxane)/(phenylmethylsiloxane)]
- poly[(dimethylsiloxane)/(diphenylsiloxane)/ (methylvinylsiloxane)];

and the following mixtures:

- the mixtures made from a polydimethylsiloxane hydroxylated at a chain end and cyclic polydimethylsiloxanes;
- the mixtures made from a polydimethylsiloxane gum and a cyclic silicone;
- mixtures of two polydimethylsiloxanes of different viscosities;
- mixtures of a polydimethylsiloxane with a polyoxyethylenated or polyoxypropylenated side chain and of a cyclic polydimethylsiloxane;

E) organopolysiloxane resins consisting of crosslinked siloxane systems containing the units $R_2SiO_{2/2}$, $RSiO_{3/2}$ or $SiO_{4/2}$, in which R denotes a hydrocarbon group containing 1 to 6 carbon atoms, or a phenyl group.

5. Use according to Claim 3, characterized in that the organopolysiloxane includes in its general structure one or more organofunctional group(s) attached directly to the siloxane chain or attached through the intermediacy of a hydrocarbon radical, and in that it is chosen from polyorganosiloxanes containing:

a) substituted or unsubstituted amine groups;
b) thiol groups;
c) carboxylate groups;
d) hydroxyalkyl groups,

and corresponding to the following formula:

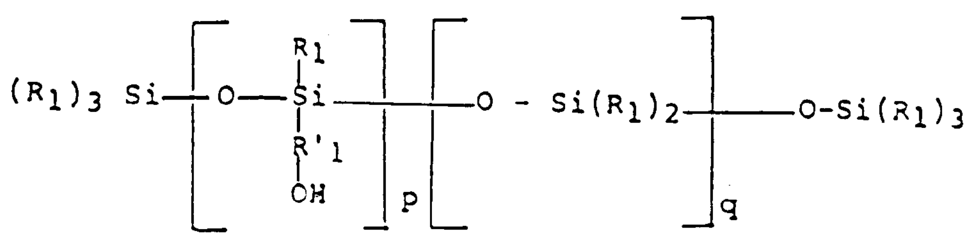

(I)

in which:

- the radicals $R_1$, which are identical or different, are chosen from methyl and phenyl radicals, at least 60 mol% of the radicals $R_1$ being methyl;
- the radical $R'_1$ is a $C_2$-$C_{18}$ divalent alkylene hydrocarbon chain unit;
- p is between 1 and 30 inclusive;
- q is between 1 and 150 inclusive;

e) alkoxylated groups;
f) acyloxyalkyl groups corresponding to the following formula:

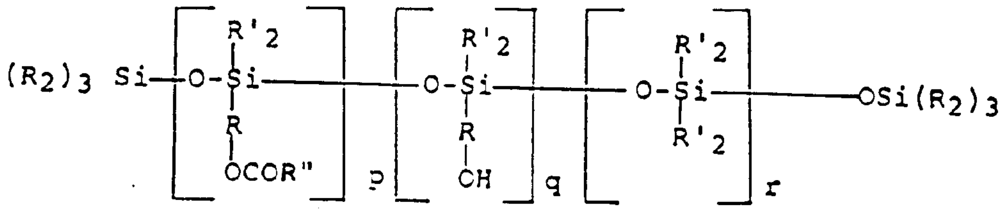 (II)

in which:

- $R_2$ denotes methyl, phenyl, $COOR_2$" [sic] or hydroxyl, only one of the $R_2$ per silicon atom may be OH;
- $R'_2$ denotes methyl, phenyl, at least 60 mol% of the set of the radicals $R_2$ and $R'_2$ are methyl;
- R" denotes $C_8$-$C_{20}$ alkyl or alkenyl;
- R denotes a linear or branched $C_2$-$C_{18}$ divalent alkylene hydrocarbon;
- r is between 1 and 120 inclusive;
- p is between 1 and 30;
- q is 0 or smaller than 0.5 p, p+q being between 1 and 30;

it being possible for the polymers of formula (II) to contain

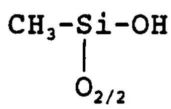

groups in proportions not exceeding 15 % of the sum p+q+r;

g) diester groups, in particular dimethyl itaconate, corresponding to the following formula:

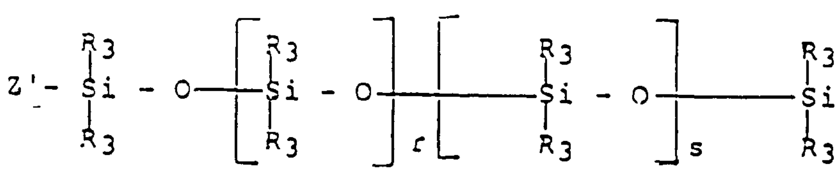 (III)

in which:

- the radicals $R_3$, which are identical or different, denote a $C_1$-$C_{20}$ alkyl, vinyl, phenyl, 3,3,3-trifluoropropyl or hydroxyl, provided that only one radical $R_3$ may denote OH per silicon atom;
- the symbols Z', which are identical or different, denote $R_3$ or Z;
- Z is chosen from the groups:

$-CH_2-CH-(COOR'_3)-CH_2-COOR'_3$

$-C(CH_3)(COOR'_3)-CH_2-COOR'_3$

- $R'_3$ denotes a $C_1$-$C_{12}$ monovalent saturated hydrocarbon radical, a $C_2$-$C_{12}$ monovalent alkoxyalkyl or a $C_6$-$C_{12}$ aryl, aralkyl or alkylaryl;
- r is between 0 and 500 inclusive;
- s is between 0 and 50 inclusive;
- if s is equal to 0, one of the symbols Z' is Z;

h) quaternary ammonium groups;

i) amphoteric or betainic groups;

j) anionic groups of alkylcarboxylic 2-hydroxyalkylsulphonate or 2-hydroxyalkylthiosulphate type.

**6.** Use according to any one of Claims 1 to 5, characterized in that the organopolysiloxane is chosen from octamethylcyclotetrasiloxane, decamethylcyclopentasiloxane, linear polyalkylsiloxanes containing trimethylsilyl end groups, mixtures of organosiloxanes and cyclic silicones, dimethyl/trimethylsiloxane resin, polymers of formula (I) in which $R_1$ denotes methyl and $R'_1$ a trimethylene or 2-methyltrimethylene group, polymers of formula (II), in which $R_2$ and $R'_2$ denote methyl, R" a mixture of $C_{16}H_{33}$ and $C_{18}H_{37}$ radicals and R denotes trimethylene or 2-methyltrimethylene; and polymers of formula (III) in which $R_3$ denotes $CH_3$ and Z' denotes OH.

7. Use according to any one of Claims 1 to 6, characterized in that the aqueous dispersion contains an ammonium acrylate/acrylamide (95/5 by weight) copolymer crosslinked with a crosslinking agent containing olefinic polyunsaturation, taken from the group consisting of divinylbenzene, tetraallyloxyethane, diallyl ether, polyallylpolyglyceryl ethers and the allyl ethers of alcohols from the sugar series.

8. Use according to any one of Claims 1 to 7, characterized in that the organopolysiloxane is present in the dispersion in proportions of between 0.5 and 50 % by weight and preferably between 1 and 30 % by weight and the ammonium acrylate/acrylamide copolymer is present in proportions of between 0.1 and 10 % by weight and preferably between 0.1 and 4 % by weight relative to the total weight of the dispersion.

9. Use according to any one of Claims 1 to 8, characterized in that the dispersion additionally contains at least one nonionic emulsifying agent and a mineral oil.

10. Use according to Claim 9, characterized in that the nonionic emulsifying agent is chosen from oil-soluble surfactants in which the hydrophile/lipophile ratio is lower than 7.

11. Use according to Claim 9 or 10, characterized in that the emulsifying agent consists of a mixture of sorbitan stearate and of a hydrophilic ethoxylated derivative.

12. Use according to Claim 9, characterized in that the mineral oil is liquid paraffin.

13. Use according to any one of Claims 9 to 12, characterized in that the nonionic emulsifying agent is present in proportions of between 0.01 and 10 % by weight and preferably between 0.01 and 0.6 % by weight, and the mineral oil is present in proportions of between 0.08 and 6.5 % by weight and preferably between 0.08 and 2.5 % by weight, the weights being defined in relation to the total weight of the dispersion.

14. Use according to any one of Claims 1 to 13, characterized in that the crosslinked ammonium acrylate/ acrylamide (95/5 by weight) copolymer is dispersed in a proportion of 30 % by weight in a water-in-oil emulsion consisting of 25 % by weight of paraffin, 4 % by weight of a mixture of sorbitan stearate and of hydrophilic ethoxylated derivative and of 41 % by weight of water.

15. Use of the aqueous dispersion according to one of Claims 1 to 14, characterized in that the water-in-oil emulsion containing the crosslinked ammonium acrylate/ acrylamide (95/5 by weight) copolymer is present in the dispersion in proportions such that the concentration of copolymer is between 0.05 and 10 % by weight and preferably between 0.1 and 5 % by weight of active substance as copolymer relative to the total weight of the dispersion.

16. Cosmetic composition in the form of aqueous dispersion intended for the treatment of hair or the skin, characterized in that the aqueous dispersion is such as defined in any one of Claims 1 to 15.

17. Composition according to Claim 16, characterized in that it additionally contains adjuvants usually employed in cosmetics, which are chosen from perfumes, colorants, preserving agents, anionic, nonionic, amphoteric or cationic surface-active agents, sequestrants, foam stabilizers, polymers, sunscreens, propellants and cosmetically active substances.

18. Composition according to either of Claims 16 and 17, characterized in that it has a pH of between 3 and 10 and preferably between 5 and 7.

19. Composition according to any one of Claims 16 to 18, intended for the treatment of hair, characterized in that it is in the form of shampoo, of rinsing product, to be applied before or after shampooing, before or after dyeing or bleaching, before or after permanent waving or straightening, or as unrinsed styling products.

20. Composition according to any one of Claims 16 to 18, intended for the treatment of the skin, characterized in that it is in the form of a bath or shower product, of a suntanning product, of an antisolar composition, of a product for shaving, of a treatment cream or milk or of a perfumed lotion.

21. Process for the cosmetic treatment of hair, characterized in that at least one composition such as defined in Claim 19 is applied to the latter.

**22.** Process for the cosmetic treatment of the skin, characterized in that a composition such as defined in Claim 20 is applied to the latter.

**23.** Dermatological composition in the form of aqueous dispersion, characterized in that the aqueous dispersion is such as defined in any one of Claims 1 to 15 and that it contains at least one dermatologically active substance in an effective quantity.

**Claims for the following Contracting State : GR**

**1.** Use of an aqueous dispersion in cosmetics, for the treatment of hair or the skin, characterized in that the said dispersion contains an organopolysiloxane and a crosslinked ammonium acrylate/acrylamide copolymer at least in a cosmetically or physiologically acceptable aqueous medium.

**2.** Use according to Claim 1, characterized in that the organosiloxane is a volatile silicone which has a boiling point of between 60°C and 260°C, chosen from:
(i) cyclic silicones containing 3 to 7 and preferably 4 to 5 silicon atoms or cyclocopolymers of the dimethylsiloxane/methylalkylsiloxane type, of formula:

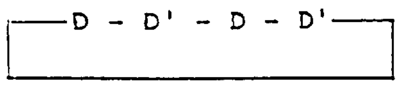

in which:
D denotes:

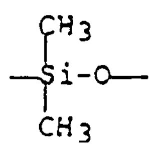

and D' denotes:

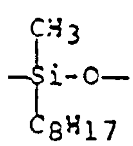

(ii) linear silicones containing 2 to 9 silicon atoms and having a viscosity lower than or equal to $5x10^{-6}$ $m^2/s$ at 25°C.

**3.** Use according to either of Claims 1 to 2, characterized in that the organopolysiloxane is a nonvolatile silicone chosen from polyalkylsiloxanes, polyarylsiloxanes, polyalkylarylsiloxanes, polyethersiloxane copolymers, modified or otherwise, silicone gums and resins, organomodified polysiloxanes and mixtures thereof.

**4.** Use according to Claim 3, characterized in that the organopolysiloxane is chosen from:
A) polyalkyl ($C_1$-$C_{20}$) siloxanes, linear polydimethylsiloxanes containing trimethylsilyl end groups, with a viscosity of $5x10^{-6}$ to $2.5x10^{-1}$ $m^2/s$ at 25°C and preferably $10^{-5}$ to 1 $m^2/s$ and linear polydimethylsiloxanes containing trihydroxysilyl end groups;
B) linear and/or branched polydimethylphenylsiloxanes and polydimethyldiphenylsiloxanes with a viscosity of $10^{-5}$ to $5x10^{-2}$ $m^2/s$ at 25°C;
C) copolymers of propylene and/or ethylene oxide with a diorganosiloxane;
D) gums with a molecular mass between 200,000 and 1,000,000, employed by themselves or in the form of a mixture in a solvent, which are chosen from the group consisting of the following copolymers:
- poly[(dimethylsiloxane)/(methylvinylsiloxane)]
- poly[(dimethylsiloxane)/(diphenylsiloxane)]

- poly[(dimethylsiloxane)/(phenylmethylsiloxane)]
- poly[(dimethylsiloxane)/(diphenylsiloxane)/ (methylvinylsiloxane)];

and the following mixtures:

- the mixtures made from a polydimethylsiloxane hydroxylated at a chain end and cyclic polydimethylsiloxanes;
- the mixtures made from a polydimethylsiloxane gum and a cyclic silicone;
- mixtures of two polydimethylsiloxanes of different viscosities;
- mixtures of a polydimethylsiloxane with a polyoxyethylenated or polyoxypropylenated side chain and of a cyclic polydimethylsiloxane;

E) organopolysiloxane resins consisting of crosslinked siloxane systems containing the units $R_2SiO_{2/2}$, $RSiO_{3/2}$ or $SiO_{4/2}$, in which R denotes a hydrocarbon group containing 1 to 6 carbon atoms, or a phenyl group.

5. Use according to Claim 3, characterized in that the organopolysiloxane includes in its general structure one or more organofunctional group(s) attached directly to the siloxane chain or attached through the intermediacy of a hydrocarbon radical, and in that it is chosen from polyorganosiloxanes containing:
   a) substituted or unsubstituted amine groups;
   b) thiol groups;
   c) carboxylate groups;
   d) hydroxyalkyl groups,
   
   and corresponding to the following formula:

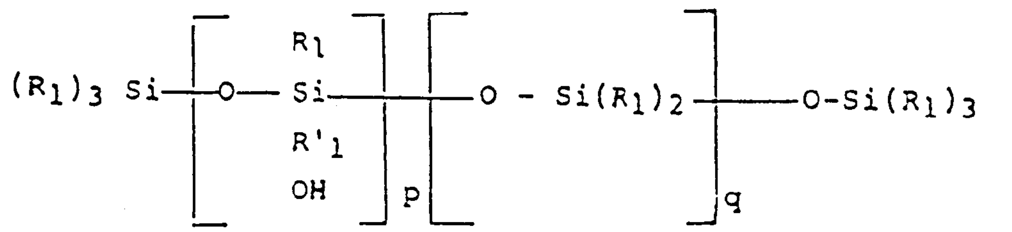

(I)

in which:

- the radicals $R_1$, which are identical or different, are chosen from methyl and phenyl radicals, at least 60 mol% of the radicals $R_1$ being methyl;
- the radical $R'_1$ is a $C_2$-$C_{18}$ divalent alkylene hydrocarbon chain unit;
- p is between 1 and 30 inclusive;
- q is between 1 and 150 inclusive;

e) alkoxylated groups;
f) acyloxyalkyl groups corresponding to the following formula:

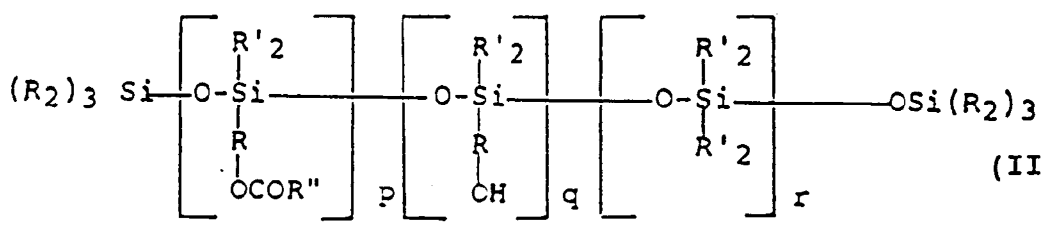

(II)

in which:

- $R_2$ denotes methyl, phenyl, $COOR_2$" [sic] or hydroxyl, only one of the $R_2$ per silicon atom may be OH;
- $R'_2$ denotes methyl, phenyl, at least 60 mol% of the set of the radicals $R_2$ and $R'_2$ are methyl;
- R" denotes $C_8$-$C_{20}$ alkyl or alkenyl;
- R denotes a linear or branched $C_2$-$C_{18}$ divalent alkylene hydrocarbon;
- r is between 1 and 120 inclusive;
- p is between 1 and 30;

- q is 0 or smaller than 0.5 p, p+q being between 1 and 30;

it being possible for the polymers of formula (II) to contain

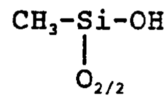

groups in proportions not exceeding 15 % of the sum p+q+r;

g) diester groups, in particular dimethyl itaconate, corresponding to the following formula:

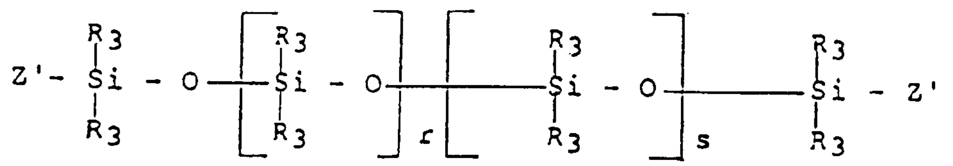 (III)

in which:

- the radicals $R_3$, which are identical or different, denote a $C_1$-$C_{20}$ alkyl, vinyl, phenyl, 3,3,3-trifluoropropyl or hydroxyl, provided that only one radical $R_3$ may denote OH per silicon atom;
- the symbols Z', which are identical or different, denote $R_3$ or Z;
- Z is chosen from the groups:

$$-CH_2-CH-(COOR'_3)-CH_2-COOR'_3$$
$$-C(CH_3)\,(COOR'_3)-CH_2-COOR'_3$$

- $R'_3$ denotes a $C_1$-$C_{12}$ monovalent saturated hydrocarbon radical, a $C_2$-$C_{12}$ monovalent alkoxyalkyl or a $C_6$-$C_{12}$ aryl, aralkyl or alkylaryl;
- r is between 0 and 500 inclusive;
- s is between 0 and 50 inclusive;
- if s is equal to 0, one of the symbols Z' is Z;

h) quaternary ammonium groups;

i) amphoteric or betainic groups;

j) anionic groups of alkylcarboxylic 2-hydroxyalkylsulphonate or 2-hydroxyalkylthiosulphate type.

**6.** Use according to any one of Claims 1 to 5, characterized in that the organopolysiloxane is chosen from octamethylcyclotetrasiloxane, decamethylcyclopentasiloxane, linear polyalkylsiloxanes containing trimethylsilyl end groups, mixtures of organosiloxanes and cyclic silicones, dimethyl/trimethylsiloxane resin, polymers of formula (I) in which $R_1$ denotes methyl and $R'_1$ a trimethylene or 2-methyltrimethylene group, polymers of formula (II), in which $R_2$ and $R'_2$ denote methyl, R" a mixture of $C_{16}H_{33}$ and $C_{18}H_{37}$ radicals and R denotes trimethylene or 2-methyltrimethylene; and polymers of formula (III) in which $R_3$ denotes $CH_3$ and Z' denotes OH.

**7.** Use according to any one of Claims 1 to 6, characterized in that the aqueous dispersion contains an ammonium acrylate/acrylamide (95/5 by weight) copolymer crosslinked with a crosslinking agent containing olefinic polyunsaturation, taken from the group consisting of divinylbenzene, tetraallyloxyethane, diallyl ether, polyallylpolyglyceryl ethers and the allyl ethers of alcohols from the sugar series.

**8.** Use according to any one of Claims 1 to 7, characterized in that the organopolysiloxane is present in the dispersion in proportions of between 0.5 and 50 % by weight and preferably between 1 and 30 % by weight and the ammonium acrylate/acrylamide copolymer is present in proportions of between 0.1 and 10 % by weight and preferably between 0.1 and 4 % by weight relative to the total weight of the dispersion.

**9.** Use according to any one of Claims 1 to 8, characterized in that the dispersion additionally contains at least one nonionic emulsifying agent and a mineral oil.

**10.** Use according to Claim 9, characterized in that the nonionic emulsifying agent is chosen from oil-soluble surfactants in which the hydrophile/lipophile ratio is lower than 7.

**11.** Use according to Claim 9 or 10, characterized in that the emulsifying agent consists of a mixture of sorbitan

stearate and of a hydrophilic ethoxylated derivative.

12. Use according to Claim 9, characterized in that the mineral oil is liquid paraffin.

13. Use according to any one of Claims 9 to 12, characterized in that the nonionic emulsifying agent is present in proportions of between 0.01 and 10 % by weight and preferably between 0.01 and 0.6 % by weight, and the mineral oil is present in proportions of between 0.08 and 6.5 % by weight and preferably between 0.08 and 2.5 % by weight, the weights being defined in relation to the total weight of the dispersion.

14. Use according to any one of Claims 1 to 13, characterized in that the crosslinked ammonium acrylate/ acrylamide (95/5 by weight) copolymer is dispersed in a proportion of 30 % by weight in a water-in-oil emulsion consisting of 25 % by weight of paraffin, 4 % by weight of a mixture of sorbitan stearate and of hydrophilic ethoxylated derivative and of 41 % by weight of water.

15. Use of the aqueous dispersion according to one of Claims 1 to 14, characterized in that the water-in-oil emulsion containing the crosslinked ammonium acrylate/ acrylamide (95/5 by weight) copolymer is present in the dispersion in proportions such that the concentration of copolymer is between 0.05 and 10 % by weight and preferably between 0.1 and 5 % by weight of active substance as copolymer relative to the total weight of the dispersion.

16. Cosmetic composition in the form of aqueous dispersion intended for the treatment of hair or the skin, characterized in that the aqueous dispersion is such as defined in any one of Claims 1 to 15.

17. Composition according to Claim 16, characterized in that it additionally contains adjuvants usually employed in cosmetics, which are chosen from perfumes, colorants, preserving agents, anionic, nonionic, amphoteric or cationic surface-active agents, sequestrants, foam stabilizers, polymers, sunscreens, propellants and cosmetically active substances.

18. Composition according to either of Claims 16 and 17, characterized in that it has a pH of between 3 and 10 and preferably between 5 and 7.

19. Composition according to any one of Claims 16 to 18, intended for the treatment of hair, characterized in that it is in the form of shampoo, of rinsing product, to be applied before or after shampooing, before or after dyeing or bleaching, before or after permanent waving or straightening, or as unrinsed styling products.

20. Composition according to any one of Claims 16 to 18, intended for the treatment of the skin, characterized in that it is in the form of a bath or shower product, of a suntanning product, of an antisolar composition, of a product for shaving, of a treatment cream or milk or of a perfumed lotion.

21. Process for the cosmetic treatment of hair, characterized in that at least one composition such as defined in Claim 19 is applied to the latter.

22. Process for the cosmetic treatment of the skin, characterized in that a composition such as defined in Claim 20 is applied to the latter.

23. Process for the preparation of a dermatological composition in the form of aqueous dispersion, characterized in that at least one dermatologically active substance is incorporated in an effective quantity in a dispersion such as defined in any one of Claims 1 to 15.

**Claims for the following Contracting State : ES**

1. Use of an aqueous dispersion in cosmetics, for the treatment of hair or the skin, characterized in that the said dispersion contains an organopolysiloxane and a crosslinked ammonium acrylate/acrylamide copolymer at least in a cosmetically or physiologically acceptable aqueous medium.

2. Use according to Claim 1, characterized in that the organosiloxane is a volatile silicone which has a boiling point of between 60°C and 260°C, chosen from:
(i) cyclic silicones containing 3 to 7 and preferably 4 to 5 silicon atoms or cyclocopolymers of the dimethylsiloxane/methylalkylsiloxane type, of formula:

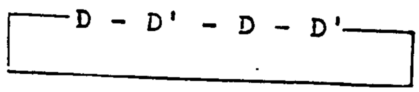

in which:

D denotes:

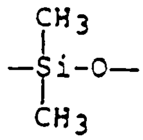

and D' denotes:

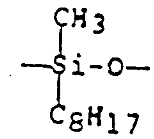

(ii) linear silicones containing 2 to 9 silicon atoms and having a viscosity lower than or equal to $5x10^{-6}$ $m^2/s$ at 25°C.

**3.** Use according to either of Claims 1 to 2, characterized in that the organopolysiloxane is a nonvolatile silicone chosen from polyalkylsiloxanes, polyarylsiloxanes, polyalkylarylsiloxanes, polyethersiloxane copolymers, modified or otherwise, silicone gums and resins, organomodified polysiloxanes and mixtures thereof.

**4.** Use according to Claim 3, characterized in that the organopolysiloxane is chosen from:

A) polyalkyl($C_1$-$C_{20}$)siloxanes, linear polydimethylsiloxanes containing trimethylsilyl end groups, with a viscosity of $5x10^{-6}$ to $2.5x10^{-1}$ $m^2/s$ at 25°C and preferably $10^{-5}$ to 1 $m^2/s$ and linear polydimethylsiloxanes containing trihydroxysilyl end groups;

B) linear and/or branched polydimethylphenylsiloxanes and polydimethyldiphenylsiloxanes with a viscosity of $10^{-5}$ to $5x10^{-2}$ $m^2/s$ at 25°C;

C) copolymers of propylene and/or ethylene oxide with a diorganosiloxane;

D) gums with a molecular mass between 200,000 and 1,000,000, employed by themselves or in the form of a mixture in a solvent, which are chosen from the group consisting of the following copolymers:

- poly[(dimethylsiloxane)/(methylvinylsiloxane)]
- poly[(dimethylsiloxane)/(diphenylsiloxane)]
- poly[(dimethylsiloxane)/(phenylmethylsiloxane)]
- poly[(dimethylsiloxane)/(diphenylsiloxane)/ (methylvinylsiloxane)];

and the following mixtures:

- the mixtures made from a polydimethylsiloxane hydroxylated at a chain end and cyclic polydimethylsiloxanes;
- the mixtures made from a polydimethylsiloxane gum and a cyclic silicone;
- mixtures of two polydimethylsiloxanes of different viscosities;
- mixtures of a polydimethylsiloxane with a polyoxyethylenated or polyoxypropylenated side chain and of a cyclic polydimethylsiloxane;

E) organopolysiloxane resins consisting of crosslinked siloxane systems containing the units $R_2SiO_{2/2}$, $RSiO_{3/2}$ or $SiO_{4/2}$, in which R denotes a hydrocarbon group containing 1 to 6 carbon atoms, or a phenyl group.

**5.** Use according to Claim 3, characterized in that the organopolysiloxane includes in its general structure one or more organofunctional group(s) attached directly to the siloxane chain or attached through the intermediacy of a hydrocarbon radical, and in that it is chosen from polyorganosiloxanes containing:

a) substituted or unsubstituted amine groups;

b) thiol groups;

c) carboxylate groups;
d) hydroxyalkyl groups, and corresponding to the following formula:

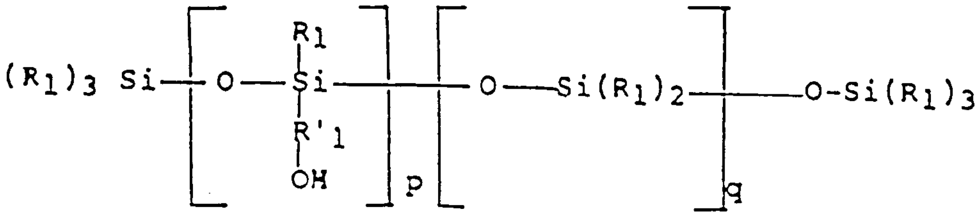

(I)

in which:

- the radicals $R_1$, which are identical or different, are chosen from methyl and phenyl radicals, at least 60 mol% of the radicals $R_1$ being methyl;
- the radical $R'_1$ is a $C_2$-$C_{18}$ divalent alkylene hydrocarbon chain unit;
- p is between 1 and 30 inclusive;
- q is between 1 and 150 inclusive;

e) alkoxylated groups;
f) acyloxyalkyl groups corresponding to the following formula:

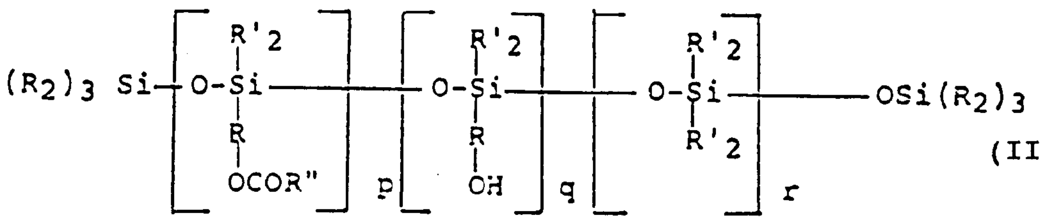

(II)

in which:

- $R_2$ denotes methyl, phenyl, $COOR_2$" [sic] or hydroxyl, only one of the $R_2$ per silicon atom may be OH;
- $R'_2$ denotes methyl, phenyl, at least 60 mol% of the set of the radicals $R_2$ and $R'_2$ are methyl;
- R" denotes $C_8$-$C_{20}$ alkyl or alkenyl;
- R denotes a linear or branched $C_2$-$C_{18}$ divalent alkylene hydrocarbon;
- r is between 1 and 120 inclusive;
- p is between 1 and 30;
- q is 0 or smaller than 0.5 p, p+q being between 1 and 30;

it being possible for the polymers of formula (II) to contain

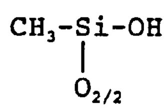

groups
in proportions not exceeding 15 % of the sum p+q+r;
g) diester groups, in particular dimethyl itaconate, corresponding to the following formula:

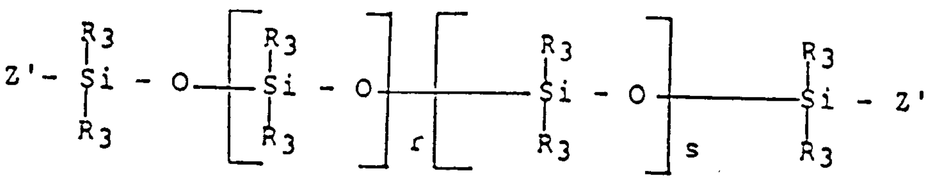

(III)

in which:

- the radicals $R_3$, which are identical or different, denote a $C_1$-$C_{20}$ alkyl, vinyl, phenyl, 3,3,3-trifluoropropyl or hydroxyl, provided that only one radical $R_3$ may denote OH per silicon atom;
- the symbols Z', which are identical or different, denote $R_3$ or Z;
- Z is chosen from the groups:

$$-CH_2-CH-(COOR'_3)-CH_3-COOR'_3$$
$$-C(CH_3)\ (COOR'_3)-CH_2-COOR'_3$$

- $R'_3$ denotes a $C_1$-$C_{12}$ monovalent saturated hydrocarbon radical, a $C_2$-$C_{12}$ monovalent alkoxyalkyl or a $C_6$-$C_{12}$ aryl, aralkyl or alkylaryl;
- r is between 0 and 500 inclusive;
- s is between 0 and 50 inclusive;
- if s is equal to 0, one of the symbols Z' is Z;

h) quaternary ammonium groups;
i) amphoteric or betainic groups;
j) anionic groups of alkylcarboxylic 2-hydroxy-alkylsulphonate or 2-hydroxyalkylthiosulphate type.

**6.** Use according to any one of Claims 1 to 5, characterized in that the organopolysiloxane is chosen from octamethylcyclotetrasiloxane, decamethylcyclopentasiloxane, linear polyalkylsiloxanes containing trimethylsilyl end groups, mixtures of organosiloxanes and cyclic silicones, dimethyl/trimethylsiloxane resin, polymers of formula (I) in which $R_1$ denotes methyl and $R'_1$ a trimethylene or 2-methyltrimethylene group, polymers of formula (II), in which $R_2$ and $R'_2$ denote methyl, R" a mixture of $C_{16}H_{33}$ and $C_{18}H_{37}$ radicals and R denotes trimethylene or 2-methyltrimethylene; and polymers of formula (III) in which $R_3$ denotes $CH_3$ and Z' denotes OH.

**7.** Use according to any one of Claims 1 to 6, characterized in that the aqueous dispersion contains an ammonium acrylate/acrylamide (95/5 by weight) copolymer crosslinked with a crosslinking agent containing olefinic polyunsaturation, taken from the group consisting of divinylbenzene, tetraallyloxyethane, diallyl ether, polyallylpolyglyceryl ethers and the allyl ethers of alcohols from the sugar series.

**8.** Use according to any one of Claims 1 to 7, characterized in that the organopolysiloxane is present in the dispersion in proportions of between 0.5 and 50 % by weight and preferably between 1 and 30 % by weight and the ammonium acrylate/acrylamide copolymer is present in proportions of between 0.1 and 10 % by weight and preferably between 0.1 and 4 % by weight relative to the total weight of the dispersion.

**9.** Use according to any one of Claims 1 to 8, characterized in that the dispersion additionally contains at least one nonionic emulsifying agent and a mineral oil.

**10.** Use according to Claim 9, characterized in that the nonionic emulsifying agent is chosen from oil-soluble surfactants in which the hydrophile/lipophile ratio is lower than 7.

**11.** Use according to Claim 9 or 10, characterized in that the emulsifying agent consists of a mixture of sorbitan stearate and of a hydrophilic ethoxylated derivative.

**12.** Use according to Claim 9, characterized in that the mineral oil is liquid paraffin.

**13.** Use according to any one of Claims 9 to 12, characterized in that the nonionic emulsifying agent is present in proportions of between 0.01 and 10 % by weight and preferably between 0.01 and 0.6 % by weight, and the mineral oil is present in proportions of between 0.08 and 6.5 % by weight and preferably between 0.08 and 2.5 % by weight, the weights being defined in relation to the total weight of the dispersion.

**14.** Use according to any one of Claims 1 to 13, characterized in that the crosslinked ammonium acrylate/acrylamide (95/5 by weight) copolymer is dispersed in a proportion of 30 % by weight in a water-in-oil emulsion consisting of 25 % by weight of paraffin, 4 % by weight of a mixture of sorbitan stearate and of hydrophilic ethoxylated derivative and of 41 % by weight of water.

**15.** Use of the aqueous dispersion according to one of Claims 1 to 14, characterized in that the water-in-oil emulsion containing the crosslinked ammonium acrylate/acrylamide (95/5 by weight) copolymer is present in the dispersion in proportions such that the concentration of copolymer is between 0.05 and 10 % by weight and preferably between 0.1 and 5 % by weight of active substance as copolymer relative to the total weight of the dispersion.

16. Process for the preparation of a cosmetic composition intended for the treatment of hair or the skin, characterized in that an aqueous dispersion such as defined in any one of Claims 1 to 15 is used.

17. Cosmetic composition in the form of aqueous dispersion intended for the treatment of hair or the skin, characterized in that the aqueous dispersion is such as defined in any one of Claims 1 to 15.

18. Composition according to Claim 17, characterized in that it additionally contains adjuvants usually employed in cosmetics, which are chosen from perfumes, colorants, preserving agents, anionic, nonionic, amphoteric or cationic surface-active agents, sequestrants, foam stabilizers, polymers, sunscreens, propellants and cosmetically active substances.

19. Composition according to either of Claims 17 or 18, characterized in that it has a pH of between 3 and 10 and preferably between 5 and 7.

20. Composition according to any one of Claims 17 to 19, intended for the treatment of hair, characterized in that it is in the form of shampoo, of rinsing product, to be applied before or after shampooing, before or after dyeing or bleaching, before or after permanent waving or straightening, or as unrinsed styling products.

21. Composition according to any one of Claims 17 to 19, intended for the treatment of the skin, characterized in that it is in the form of a bath or shower product, of a suntanning product, of an antisolar composition, of a product for shaving, of a treatment cream or milk or of a perfumed lotion.

22. Process for the cosmetic treatment of hair, characterized in that at least one composition such as defined in Claim 20 is applied to the latter.

23. Process for the cosmetic treatment of the skin, characterized in that a composition such as defined in Claim 21 is applied to the latter.

24. Process for the preparation of a dermatological composition, characterized in that an aqueous dispersion such as defined in any one of Claims 1 to 15 is used and that an effective quantity is incorporated therein of at least one dermatologically active substance.